# FASCICULE DE BREVET EUROPEEN

(11) **EP 3 521 314 B1**
(45) Date de publication et mention de la délivrance du brevet: **24.02.2021**
(21) Numéro de dépôt: 19164117.4
(22) Date de dépôt: 11.07.2014
(51) Int. Cl.: C07K 16/30, C07D 207/456

(54) **CONJUGUES ANTICORPS-MEDICAMENT ET LEUR UTILISATION EN THERAPIE**
ANTIKÖRPER-WIRKSTOFF-KONJUGATE UND IHR THERAPEUTISCHER EINSATZ
ANTIBODY-DRUG CONJUGATES AND USE THEREOF IN THERAPY

(30) Priorité: 11.07.2013 FR 1356837
(43) Date de publication de la demande: 07.08.2019
(62) Demande divisionnaire de: 14745210.6
(73) Titulaire: Mc SAF, 37000 Tours (FR)
(72) Inventeur: JOUBERT, Nicolas, 37550 SAINT AVERTIN (FR); VIAUD-MASSUARD, Marie Claude, 37100 TOURS (FR); RESPAUD, Renaud, 37000 TOURS (FR)
(74) Mandataire: Cabinet Beau de Loménie

(56) Documents cités:
- WO-A1-2013/085925

## Description

La présente invention concerne des conjugués anticorps-médicament et leur utilisation en thérapie, notamment en thérapie anti-cancéreuse ou anti-inflammatoire. La présente invention concerne également des produits de synthèses utiles comme linker, composés d'une tête de linker et d'un corps de linker. La présente invention concerne également un procédé de préparation des linkers et des conjugués anticorps-médicament.

Un conjugué anticorps-médicament est une nouvelle forme de médicament, notamment utilisé comme anticancéreux ayant une action ciblée. L'anticorps thérapeutique se lie à un ou plusieurs types cellulaires en fonction de sa spécificité et libère classiquement dans les cellules, un agent cytotoxique.

Un conjugué anticorps-médicament constitue un moyen de délivrance sélective d'un agent cytotoxique. Cette ingénierie permet donc de combiner la spécificité du ciblage par les anticorps avec de nouvelles fonctions effectrices puissantes par les agents qui leur sont conjugués.

A ce jour, plus de 20 conjugués anticorps-médicaments sont en développement (Senter, P. D. et al. Annu. Rev. Med. 2013, 64, 15-29).

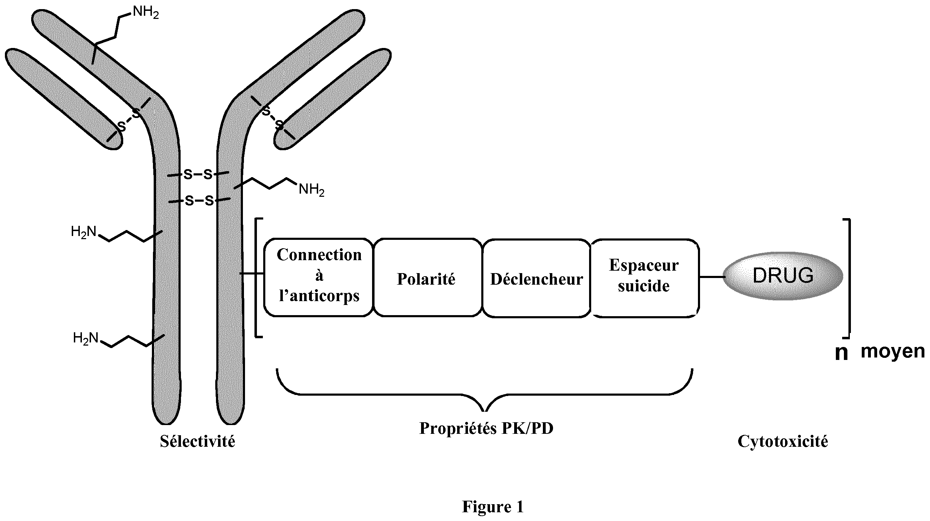

La structure générale d'un conjugué anticorps-médicament est celle décrite sur la figure 1.

Le produit liant l'anticorps et le médicament est appelé agent de liaison ou linker. Il peut être greffé sur l'anticorps via l'une au moins des huit cystéines formant les 4 ponts disulfure inter-chaînes ou sur l'une au moins des huit lysines.

Le nombre de molécules de médicaments greffées sur l'anticorps détermine un ratio appelé Drug-Antibody Ratio (DAR).

Après fixation sur son antigène cible, l'anticorps est internalisé dans la cellule par endocytose médiée par des récepteurs. Les vésicules fusionnent avec des lysosomes où la molécule cytotoxique est libérée de l'anticorps via différents mécanismes. L'agent cytotoxique actif agit ensuite directement sur la cellule en induisant sa mort et parfois sur les cellules cancéreuses voisines par transport ou diffusion dans l'environnement.

L'anticorps est donc principalement utilisé comme vecteur et apporte l'agent cytotoxique dans la cellule.

On entend par « agent cytotoxique », une molécule capable d'inhiber ou d'empêcher la fonction d'une cellule.

L'agent cytotoxique fixé à l'anticorps est une prodrogue qui devient active dans la cellule tumorale après libération (Jaracz *et al.* 2005). L'activité de la molécule libre doit être suffisante pour tuer la cellule cancéreuse, même à faible concentration.

Malgré leur succès grandissant, les conjugués anticorps-médicaments présentent un inconvénient notable, du fait qu'ils sont plus complexes et ont une structure hétérogène comparé à l'anticorps original correspondant. L'attachement d'un agent cytotoxique par l'intermédiaire d'un linker influence significativement la pharmacocinétique et la pharmacodynamique de l'anticorps (PK-PD) et donc l'index thérapeutique.

Les conjugués anticorps-médicaments ont préférentiellement un DAR moyen égal à 4 pour une activité optimale mais ce ratio peut varier de 1 à 13, sachant que pour un DAR il peut exister une population d'entités distinctes.

Beaucoup de chercheurs travaillent sur cette hétérogénéité. Des essais visant à modifier la séquence de l'anticorps, par introduction d'acides aminés naturels ou non naturels, ont été menées (Mallet, W. et al. Nat. Biotechnol. 2008, 26, 925-932 et Schultz, P. G. et al. Proc. Natl. Acad. Sci. 2012, 109, 16101-16106). Néanmoins, cette méthode présente un désavantage, celui de devoir être transposée pour chaque anticorps d'intérêt, ce qui nécessite un travail conséquent sur l'anticorps de départ.

En conséquence, le problème technique qui se dégage de l'art antérieur réside dans le fait d'obtenir un moyen technologique permettant de greffer de manière contrôlée un agent cytotoxique sans modification de la séquence de l'anticorps d'intérêt, un moyen technologique adaptable à n'importe quel anticorps.

La présente invention repose sur un produit caractérisé en ce qu'il répond à la formule I choisie parmi les formules IB2 et IA2: 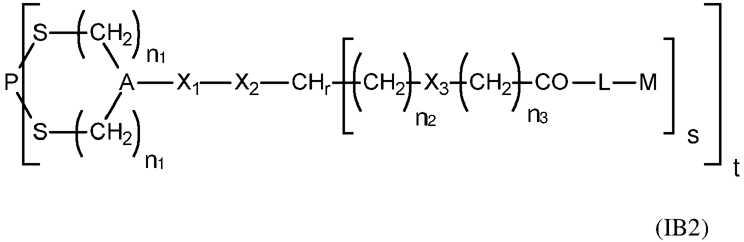 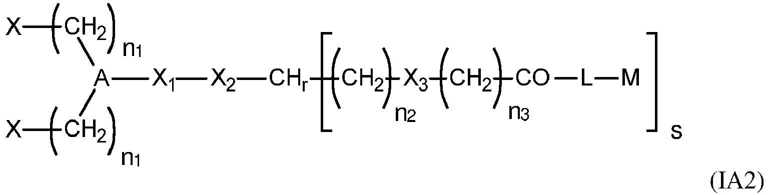 formules IA2 et IB2 dans lesquelles :
le groupe : 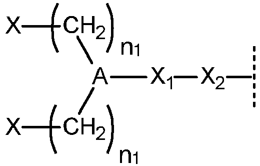 est choisi parmi les radicaux : 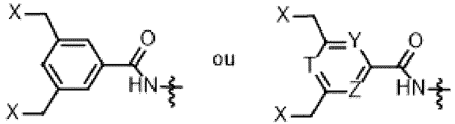 et en ce que le groupe : 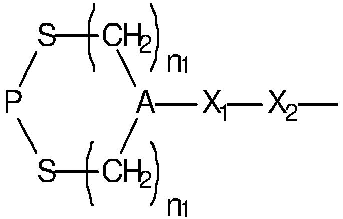 est choisi parmi les radicaux : 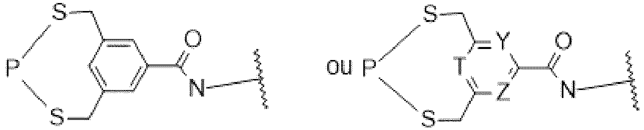
X est Br ou I,
dans la triade d'atomes (T,Y,Z) chacun de ces atomes représente indépendamment des deux autres atomes, un atome de carbone ou d'azote, en particulier T est un atome d'azote et Y et Z représentent CH,
X₃ est un oxygène ou une simple liaison
s est égal à 1, 2 ou 3
r est égal à 0, 1 ou 2
étant entendu que la somme r+s est égale à 3
n₂ est un entier égal à 1, 2 ou 3 et n₃ est un entier égal à 1, 2 ou 3 - L-M est un radical dans lequel, L représente un corps de linker et M est un médicament cytotoxique choisi parmi un agent chimiothérapeutique ou une toxine,
P est une protéine comprenant au moins un pont disulfure et
t représente un entier de 1 à 15,
ainsi que les dérivés de la ou des fonctions acides carboxyliques choisis parmi les sels, les esters ou les amides.

P est une protéine comprenant au moins un pont disulfure et t représente un entier de 1 à 15, de préférence de 1 à 6 et 13.

Au sens de la présente description, on entend par « nucléofuge », tout ion ou substituant ayant la capacité de se détacher d'une molécule.

Les sels sont choisis dans le groupe comprenant les sels alcalins, notamment de sodium ou de potassium, les sels de lithium, de magnésium, calcium et baryum, les sels d'ammonium, les sels d'amines, les sels d'aminoalcools tels la triéthanolamine ou la monoéthanolamine.

Les esters d'acide carboxylique sont formés de préférence avec les alkyls linéaires ou ramifiés ayant de 1 à 6 atomes de carbone, de préférence le tert-butyle.

Parmi les amides d'acide carboxylique, l'amide formée avec le butanimide est préféré.

Au sens de la présente description, on entend par « fonction réactive terminale », tout atome ou groupe d'atomes permettant un ou plusieurs assemblages moléculaires par chimie organique classique ou chimie combinatoire.

Dans un autre mode de réalisation particulier de l'invention, le produit selon l'invention comprend un corps de linker L répondant à la formule générale III, IIIa ou IIIb ci-dessous : 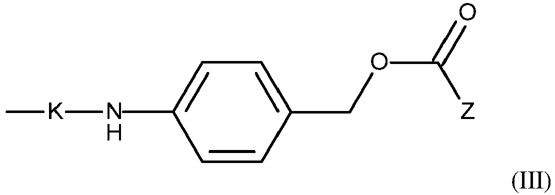 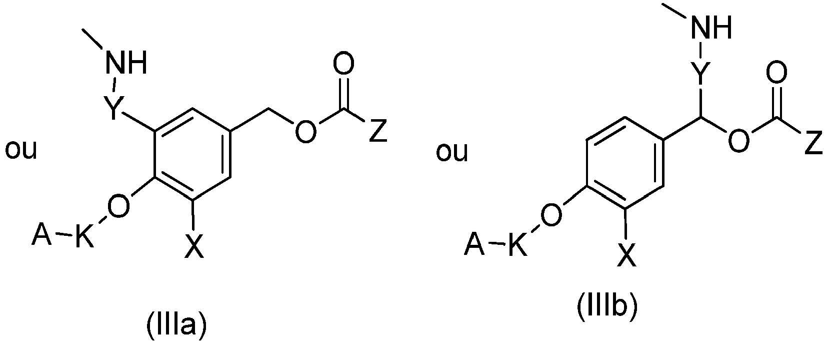
dans lesquelles K représente un radical (AA)_{n₅} représentant une succession de n5 acides aminés identiques ou différents, naturels ou non naturels ou K représente un site de clivage reconnu par une enzyme ou K représente un radical hydrazino éventuellement couplé au radical (AA)_{n₅},
soit K représente un groupe saccharide choisi de préférence parmi un acide bêta-glucuronique ou un bêta-D-galactose ou un beta-D-glucose ou un alpha-D-mannose ou un N-acétyl-D-glucosaminyle ou un N-acétyl-D-galactosaminyle, un D-glucuronyle, un L-iduronyle, un D-glucopyranosyle, un D-galactopyranosyle, un D-mannopyranosyle ou L-fucopyranosyle.
X représente un hydrogène ou un groupement NO₂,
A représente de préférence un atome d'hydrogène,
Y représente un enchaînement 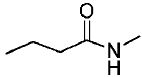 ou une simple liaison ou un bras-espaceur chimique choisi de préférence dans le groupe formé par les radicaux alkyle linéaire ou ramifiée ayant de 1 à 30 atomes de carbone éventuellement interrompus par un ou plusieurs atomes d'oxygène de soufre ou d'azote, comme par exemple un polyéthylène glycol.
Z représente un groupe partant, et n₅ représente un entier de 1 à 6.

Le groupe partant Z peut être choisi dans le groupe comprenant le para nitro ou para cyano phényloxy. De préférence, le groupe partant Z est l'un des radicaux : 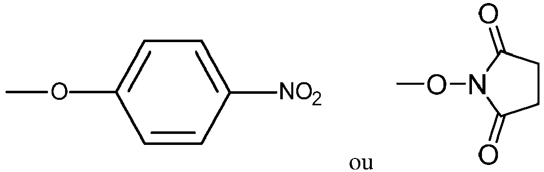 ou un atome de chlore.

Dans un autre mode de réalisation de l'invention, le produit répondant à la formule générale

III, ou IIIa ou IIIb comprend le radical (AA)_{n₅}, lequel est tel que n₅ est égal à 1, 2, 3, 4 ou 5, et AA est un couple d'acides aminés, lesdits acides aminés étant choisi dans le groupe constitué de : une valine, une citrulline, une phénylalanine, une lysine, un acide aspartique, une méthionine, une asparagine, une proline, une isoleucine, une alanine, une arginine, une glycine ou un acide glutamique.

Dans un mode de réalisation préféré, n₅ est égal à 2 ou 3, de préférence 2 et (AA)_{n₅} est un couple d'acides aminés choisi parmi : une valine et une citrulline, ou une phénylalanine et une lysine, ou une valine et une alanine, ou une valine et un acide aspartique, ou une lysine et une méthionine, ou une lysine et une asparagine, ou une proline et une isoleucine, ou une proline et une lysine, ou une lysine et une méthionine, ou une lysine et une asparagine, ou une valine et une lysine, ou une alanine et une lysine, ou une phénylalanine et une lysine, ou deux phénylalanines et une lysine, ou une alanine, ou une phénylalanine et une lysine, ou deux arginines, ou une lysine et une arginine, ou un acide glutamique, ou une glycine et une arginine, ou deux glycines et une arginine.

Dans un mode particulier de réalisation de l'invention, le produit répondant à la formule III, ou IIIa, ou IIIb, comprend un élément K qui représente un site de clivage par une enzyme choisie parmi les enzymes de type cathepsine B, cathepsine C, cathepsine D, les enzymes choisies parmi la plasmine, une protéase lysosomale, une enzyme lysosomale, ou l'urokinase plasminogen activator (uPA), élastase, protéinase 3, cathepsine G.

K peut aussi représenter un site de reconnaissance de clivage par une enzyme de type métalloprotéinase matricielle (MMP). Dans ce cas, l'enzyme type métalloprotéinase matricielle (MMP) est choisie de préférence parmi les collagénases 1, 2 et 3 (MMP-1, MMP-8, MMP-13), les gélatinases A et B (MMP-2 et MMP-9), les stromélysines 1 et 2 (MMP-3 et MMP-10), les matrilysines 1, 2 et 3 (MMP-7, MMP-26, MMP-11), l'émastase élastase des macrophages (MMP-12), les MMP membranaires (MMP-14, MMP-15, MMP-16 MMP-17, MMP-24 et MMP-25), l'énamélysine (MMP-20), la CA-MMP (MMP-23), l'épilysine (MMP-18) et la PSMA.

K peut également représenter un site de reconnaissance de clivage par une estérase, une carboxylestérase, les alkalines phosphatases, les protéases, les peptidases, les cathepsines, la glucosidase, la galactosidase, la β-D-galactosidase, l'induronidase, la β-glucuronidase, la mannosidase, la *N*-acétyl-D-glucosaminidase ou par la *N*-acétyl-D-galactosaminidase.

Dans le cadre de la présente invention, on entend par « site de clivage », la position où a lieu la coupure de la chaîne peptidique par une enzyme, par exemple le site Valine-Citrulline, les sites de clivage K ci-dessus ou la position où a lieu la reconnaissance enzymatique.

Au sens de la présente description, on entend par « médicament cytotoxique », toute molécule naturelle ou de synthèse, capable d'inhiber ou d'empêcher la fonction des cellules. On entend également par « cytotoxique », la propriété qu'un agent chimique ou biologique, d'altérer des cellules, éventuellement jusqu'à les détruire.

Dans un mode de réalisation particulier de l'invention, le médicament M est un médicament choisi parmi tout composé ayant obtenu une AMM et qui est utilisé en thérapie anti-cancéreuse ou anti-inflammatoire, toute molécule en cours d'évaluation clinique en thérapie anti-cancéreuse ou anti-inflammatoire. Le médicament M sera choisi par exemple parmi le paclitaxel (Taxol®) ou le docetaxel (Taxotère®) ou l'un de ses dérivés, le topotecan, le bortezomib, la daunorubicine, les analogues de daunorubicine, la vincristine, la mitomycine C, l'acide rétinoïque, le méthotrexate, l'ilomédine, l'aspirine, un IMIDs, le lénalidomide, le pomalidomide.

Dans un autre mode de réalisation particulier de l'invention, le médicament est M est sélectionné dans le groupe constitué de la duocarmycine et ses analogues, les dolastatines, la combretastatine, la calichéamicine, la N-acétyl-γ-calichéamycine (CMC), un dérivé de calichéamycine, la maytansine et ses analogues, DM-I, l'auristatine E, l'auristatine EB (AEB), l'auristatine EFP (AEFP), la monométhyle d'auristatine E (MMAE), la monométhyle d'auristatine F (MMAF), la tubulysine, le disorazole, les epothilones, l'echinomycine, l'estramustine, la cemadotine, l'eleutherobine, la methopterine, l'actinomycine, la mitomycin A, la camptothécine, un dérivé de la camptothécine, le SN38, la maytansine, un dérivé de type maytansinoïde, le DM1, le DM4, le TK1, l'amanitin, une pyrrolobenzodiazépine, un dimère de pyrrolobenzodiazépine, une pyrrolopyridodiazépine, un dimère de pyrrolopyridodiazépine, un intercalant de l'ADN, un inhibiteur d'histone deacetylase, ou un inhibiteur de tyrosine kinase.

Dans un autre mode de réalisation particulier de l'invention, le médicament M est sélectionné parmi l'exotoxine de pseudomonas (PE), la deBouganin, la Bouganin, la toxine diphtérique (DT) et la ricine.

Dans un autre mode de réalisation particulier de l'invention, le produit selon l'invention répond à la formule IB2 ci-dessous : 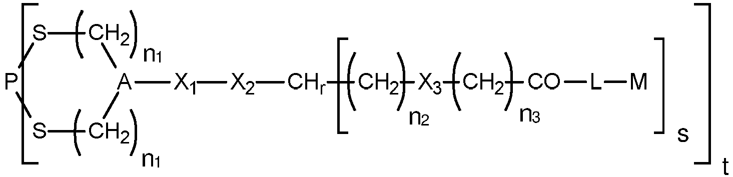 tel que défini précédemment.

Dans un mode particulier de réalisation de l'invention, la protéine P comprenant au moins un pont disulfure est sélectionnée parmi un agoniste ou un antagoniste, un anticorps ou un fragment d'anticorps, une protéine de fusion comportant un anticorps ou un fragment d'anticorps, une albumine ou un peptide.

On entend par « agoniste », toute molécule qui en se liant spécifiquement à un récepteur cellulaire donné, déclenche les mêmes effets que le ligand naturel.

On entend par « antagoniste », toute molécule qui en se liant spécifiquement à un récepteur cellulaire donné, peut ainsi bloquer l'action du ligand endogène en s'opposant à la liaison du ligand à son récepteur.

On entend par « anticorps », également couramment appelé « immunoglobuline », un hétérotétramère constitué de deux chaînes lourdes d'environ 50-70 kDa chacune (dites les chaînes H pour Heavy) et de deux chaînes légères d'environ 25 kDa chacune (dites les chaînes L pour Light), liées entre elles par des ponts disulfures intra et intercaténaires. Chaque chaîne est constituée, en position N-terminale, d'une région ou domaine variable, dit VL pour la chaîne légère, VH pour la chaîne lourde, et en position C-terminale, d'une région constante, constitué d'un seul domaine dit CL pour la chaîne légère et de trois ou quatre domaines nommés CH1, CH2, CH3, CH4, pour la chaîne lourde.

On entend par « fragment d'anticorps », toute partie d'une immunoglobuline obtenue par digestion enzymatique ou obtenue par bioproduction.

L'anticorps selon la présente invention peut-être un anticorps monoclonal chimérique, humanisé ou humain, monospécifique ou bispécifique.

Par « anticorps chimérique », on entend un anticorps dont les séquences des régions variables des chaînes légères et des chaînes lourdes appartiennent à une espèce différente de celle des séquences de régions constantes des chaînes légères et des chaînes lourdes.

Aux fins de l'invention, les séquences des régions variables des chaînes lourdes et légères sont préférentiellement d'origine murine tandis que les séquences des régions constantes des chaînes lourdes et légères appartiennent à une espèce non-murine. A cet égard, pour les régions constantes, toutes les espèces de mammifères non-murins sont susceptibles d'être utilisées, et en particulier l'homme, le singe, les suidés, les bovidés, les équidés, les félidés, les canidés ou encore les oiseaux, cette liste n'étant pas exhaustive.

De façon préférée, les anticorps chimériques selon l'invention contiennent des séquences de régions constantes des chaînes lourdes et légères d'origine humaine et les séquences de régions variables des chaînes lourdes et légères d'origine murine.

Par « anticorps humanisé », on entend un anticorps dont tout ou partie des séquences des régions impliquées dans la reconnaissance de l'antigène (les régions hypervariables ou CDR : Complementarity Determining Region) et parfois certains acides aminés des régions FR (régions Framework)) sont d'origine non-humaine tandis que les séquences des régions constantes et des régions variables non-impliquées dans la reconnaissance de l'antigène sont d'origine humaine.

Par «anticorps humain», on entend un anticorps contenant uniquement des séquences humaines, aussi bien pour les régions variables et constantes des chaînes légères que pour les régions variables et constantes des chaînes lourdes.

Dans un autre mode de réalisation particulier de l'invention, la protéine P peut être un fragment d'anticorps. Ce fragment d'anticorps est sélectionné parmi le groupe constitué de : Fab, F(ab)'2, Fc, F'c, pFc', ScFv, Fv, Fd, Fabc, diabody, minibody, ScFv-Fc ou ScFv-Fv.

La digestion enzymatique des immunoglobulines par la papaïne génère deux fragments identiques, qu'on appelle les fragments Fab (Fragment antigen binding), et un fragment Fc (Fragment cristallisable).

La digestion enzymatique des immunoglobulines par la pepsine génère un fragment F(ab')2 et un fragment Fc scindé en plusieurs peptides.

F(ab')2 est formé de deux fragments Fab' liés par des ponts disulfures intercaténaires.

Les parties Fab sont constituées des régions variables et des domaines CH1 et CL, tandis que la région Fc est constituée des deux domaines globulaires CH2 et CH3 (et CH4 quand il est présent).

Le fragment Fab' est constitué de la région Fab et d'une région charnière. Fab'-SH fait référence à un fragment Fab' dans lequel le résidu cystéine de la région charnière porte un groupe thiol libre (Carter et al., Nature BioTechnology 10:163-167 (1992)). Le fragment Fv est composé des domaines VH et VL liés par des ponts disulfures. C'est le plus petit fragment conservant l'activité de liaison à l'antigène.

Le fragment Fd est formé des domaines VH et CH1.

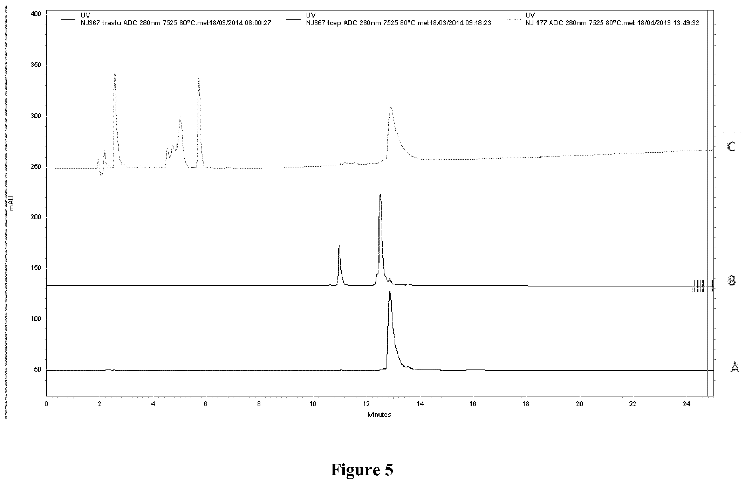

Les fragments « F'c », « pFc' », et « Fabc » sont définis dans les figures 5A à 5C.

Le scFv (single chain Fragment variable) est un fragment issu de l'ingénierie des protéines qui est constitué uniquement des domaines variables VH et VL. La structure est stabilisée par un court bras peptidique flexible, appelé linker, qui est placé entre les deux domaines.

Le fragment ScFv peut être lié à un fragment Fc pour donner un ScFv-Fc ou un fragment Fv pour donner un ScFv-Fv.

Si on réduit la taille du peptide de liaison, de nouvelles contraintes stériques apparaissent entre les scFv, les domaines VH et VL ne peuvent plus s'associer en une structure fonctionnelle et on obtient des structures multimériques (dimérique : « diabody », trimérique : « triabody » et tétramérique : « tétrabody »).

Dans le cadre de la présente invention, les diabody peuvent être utilisés. Ils peuvent avoir des valences et des spécificités multiples.

Le terme « valent » dénote la présence d'un nombre déterminé de sites de liaison à l'antigène dans une molécule d'anticorps.

Le terme « spécifique » fait référence aux types différents d'antigènes pouvant se lier sur le même anticorps.

Ainsi, dans la présente invention, on entend par « diabody » un dimère de scFv, ledit diabody étant bivalent, mono ou bispécifique selon qu'il fixe deux antigènes identiques ou différents. Les fragments Fab, Fv et scFv sont monovalents et monospécifiques.

On entend par « minibody », un fragment composé d'une chaine légère, d'une chaine lourde lié à un groupement CH3.

Dans le cadre de la présente invention, l'anticorps ou le fragment d'anticorps est dirigé contre un antigène de tumeur ou d'inflammation.

Dans un mode de réalisation particulier de l'invention, l'anticorps ou le fragment d'anticorps est dirigé contre l'un des antigènes du cluster de différentiation (CD), dont le numéro d'identification varie entre le CD1a et le CD363 ; dans cette liste, les CD suivants sont préférés : CD1a, CD363, CD3, CD4, CD13, CD19, CD20, CD21, CD22, CD25, CD30, CD31, CD33, CD34, CD37, CD39, CD40, CD44, CD47, CD52, CD56, CD66e, CD70, CD72, CD73, CD74, CD79, CD79b, CD80, CD86, CD117, CD138, CD194, CD205, CD227 ou CD248. L'anticorps ou le fragment d'anticorps peut également être dirigé contre l'un des antigènes choisis dans la liste formée par les suivants : CA125, G250, GD2, HLA-DRβ, MUC1, VEGF, VEGFA, VEGF-R1/2, TRAIL-R2 (DR5), EpCAM, GPIIb, GPIIIa, TNF alpha, TNFR, TNT, Lewis Y, EGFR, HER-2, HER-3, HER-3 MM-111, HER-4, homodimère ou hétérodimère entre des membres de la famille erbbn (n entre 1 et 4), AXL, Protéine F, IgE-Fc, VEGF-A, intégrine α4, intégrine α4β7, intégrine αV, C5, IL-6R, IL-6Rα, IL12, IL15, IL18, IL23, IL-1β, IL-1, TPO-R, GPNMB, PSMA, PSA, PAP, PSM, intégrine αv, Cripto, TACSTD2 (TROP2 ou EGP1), CEA, Folate receptor 1, Mucin 16, Endothelin Receptor ETB, STEAP1, SLC44A4 (AGS-5), Nectin 4, AGS-16, Guanylyl cyclase C, Mucin 1, EGFRvIII, Mesothelin, IL2R, A33, Can, VEGF-B, VEGF-C, VEGF-D, VEGF-E, VEGFR-1, VEGFR-2, VEGFR-3, TGFβ, TGFβR, FGF, FGF8b, FGFR, PDGF, PDGFR, PDGFRα, PDGFRβ, Ang-1, Ang-2, intégrine, αvβ3, αvβ5, α3β1, α6β4, α2β1, anti-intégrine α4, RANK-L, BLyS, c-MET, DR, DR10, TCRαβ, ICOS, CTLA-4, CAIX (MN), EphA2, CA6, CA6 de l'ovaire, CA6 cervical, CA6 du sein, angiopoiétine-2, Cripto, ENPP3, Mesothelin, FOLR1, Nectin-4, TIM-1, Muc-16, Tissue Factor, LIV-1, GM2, α 5 integrin, TLR-7, PD-1, AFP, CA125 (MUC16), Sialyl LewisY, CAMPATH-1, HLA-DR, anti-idiotype, carcinoembryonic antigen (CEA), TAG-72, Folate-binding protein, A33, G250, gangliosides (incluant GD2, GD3, GM2), LeY, collagen 4 (collagen IV), collagen 18 (collagen XVIII), SC6, CA-125, CA19-9, p185HER2, de2-7 EGFR, Fibroblast activation protein (FAP), Tenascin, metalloproteinases, Endosialin, Carbonic anhydrase, Galectin 9, Aldolase A, eIFγ4, Tyrosinase, Galectin 4, HERKV-K10, p53, NY-LU-12, Restin, NY-CO-38, MAGE-1, MAGE-4a, SSX2, NY-ESO-1, SCP-1, HGFR, PTK 7, CCK-4, PDGFR, PTP-LAR, CDCP1, CADM1, IGSF4, Lu, BCAM, CEACAM6, JAM-A, PTGFRN (CD9P-1), MCAM, MUC18, MCP, EMMPRIN, TfR, TRAILR2, C1qR, hTERT, Survivin, MDM2, CYP1B1, Melan-A, MART-1, MART-2, Melanosomal proteins, gp100, neo-PAP, CDC27, MAGEs, WT1, MUM-1, MUM-2, MUM-3, BRAF, TPI, fibronectin, K-ras, β-catenin, CDK4, caspase-8, p14ARF, p16INK4a, TGFβRII, bcr-abl, SYT-SSX, TRP-1, TRP-2, GnT-V, tyrosinase, FGF5, TEL-AML1, Proteinase 3, HER2/neu, AFP, MUC-1, EBV-EBNA, HTLV-1 tax, HPV16-E7, mutated HLA-A2, HA1, SART3, GnT-V, CEACAM5, AGS-16, GPNMB, ESAT-6, RANK, CanAg, fibrin, TF, PRAME, CA19-9, CA50, CA125, CA195, CAM17.1/WGA, AFP, β2-MG DU-PAN2, HE4, b-2 microglobulin, transferrin, transthyretin, ApoA1, TROP-2, CTLA-4, GITR, PD-1, PD-L1, c-KIT, CD11b-CD18 integrin heterodimer, DNA/Histon H1, Folate, EpCAM, Tenascin-c, ECM (proteoglycan ou fibronectin), fibrinogen, SV40 large T antigen, SC6-Ag, SC-Ag, DR4 (death receptor 4), DR5, ESA, mucin, hPAM4, hRS7, HLA-DR, CCR4, MTX1, MTX2, PECAM, thrombomoduline Tn, cathepsin D, TYRO-3, MER. L'anticorps ou le fragment d'anticorps peut également être dirigé contre un complexe PF4/héparine.

La description concerne également un procédé de préparation des produits de formules I, caractérisés en ce que, si désiré, l'on fait agir un produit de formule IA3 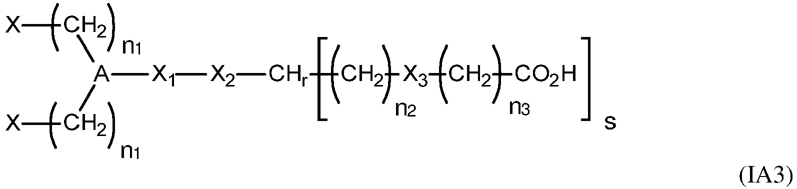 ainsi que les dérivés de la ou des fonctions acides carboxyliques tels que les sels, les esters ou les amides de préférence l'amide formée avec le butanimide,
formule IA3 dans laquelle :
X est Br ou I, ou un groupe 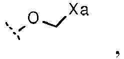 ou un groupe 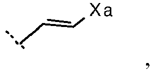 lequel Xₐ est un halogène ou un nucléofuge
A représente ou bien un radical aryle ou cycloalkyle, lesdits radicaux aryles ou cycloalkyles étant carbocycliques ou hétérocycliques,
ou bien A représente un groupe 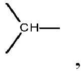 un groupe 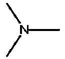 ou un groupe 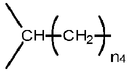
X₁ est un C=O ou un groupe NH ou une simple liaison
X₂ est un groupe NH ou un C=O ou une simple liaison
X₃ est un oxygène ou une simple liaison s est égal à 1, 2 ou 3
r est égal à 0, 1 ou 2
étant entendu que la somme r+s est égale à 3
n₁ est un entier égal à 0 ou 1 ; n₂ est un entier égal à 1, 2 ou 3 et n₃ est un entier égal à 1, 2 ou 3,
n₄ est un entier égal à 1, 2, 3, ou 4,
avec un produit de formule H2N - L, ou HO - L dans laquelle L représente un corps de linker comportant une fonction réactive terminale, pour obtenir un produit de formule IA1 : 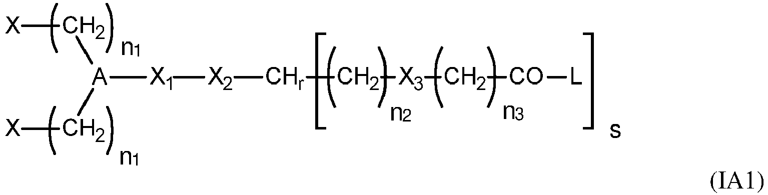 produit de formule IA1 que, si désiré, l'on fait réagir avec un produit de formule :
H2N - M, ou HO - M dans laquelle M représente un médicament cytotoxique choisi parmi un agent chimiothérapeutique ou une toxine, pour obtenir un produit de formule IA2 : 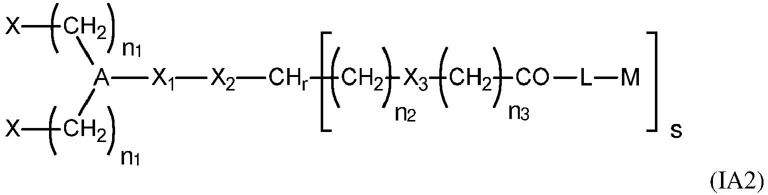 ou produit de formule IA3 que, si désiré, l'on fait agir avec un reste d'un radical fluorescent - FL pour obtenir un produit de formule (IA4) 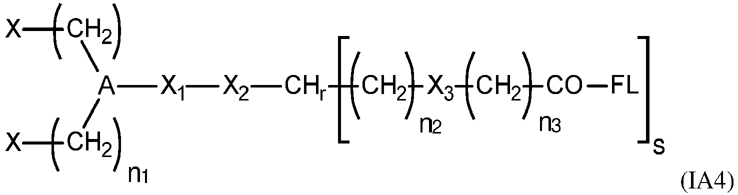 ou produit de formule IA3 que, si désiré, l'on fait agir avec un reste d'un radical radioactif - R* pour obtenir un produit de formule (IA5)

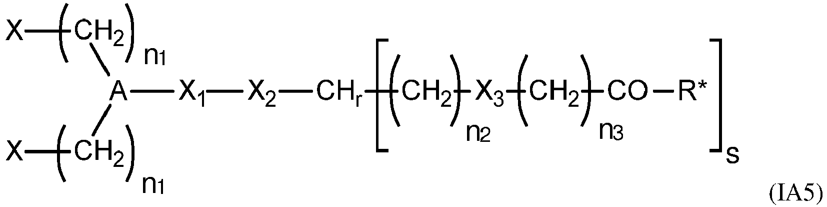

Les produits de formules IA1 à IA5 correspondant aux produits de formule IA que, si désiré, l'on fait réagir avec un produit de formule P, P étant une protéine comprenant au moins un pont disulfure et t représente un entier de 1 à 15, de préférence de 1 à 6 et 13, pour obtenir un produit de formule (IB) : 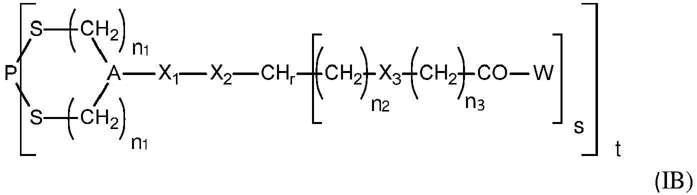 dans laquelle W représente
- un radical - L qui représente un corps de linker comportant une fonction réactive terminale,
- un groupe fluorophore - FL utilisé en diagnostique ou analyse, impliquant une détection de fluorescence choisi de préférence parmi la rhodamine ou l'un de ses dérivés, de préférence la rhodamine B, l'isocyanate de fluorescéine (FITC), une Cy Dye choisie de préférence parmi Cy5, Cy5.5, Cy7, une Alexa fluor Dye choisie de préférence parmi Alexa Fluor 647, 700 ou 750, le Texas Red, le Nile Blue A, l'allophycocyanin (APC) et ses conjugués avec d'autres fluorochromes notamment ceux précédemment cités, la phycoerythrin (PE) et ses conjugués avec d'autres fluorochromes notamment ceux précédemment cités,
- un radical R* radioactif qui contient de préférence
   soit un ligand bifonctionnel, éventuellement bimodal, choisi de préférence parmi les dérivés de DOTA, DTPA, C-DOTA, NODAGA, NETA, C-NETA, DEPA, C-DEPA, TETA, TE2A, HYNIC, DAT, MAMA,
   soit un ligand bifonctionnel choisi de préférence parmi l'un des chélateurs acycliques de la liste constituée par EDTA, CyEDTA, EDTMP, DTPMP, DTPA, CyDTPA, Cy₂DTPA, DTPA-MA, DTPA-BA, BOPA,
   soit un ligand bifonctionnel choisi de préférence parmi l'un des chélateurs macrocycliques de la liste constituée par DOTA, TRITA, TETA, DOTA-MA, DO3A-HP, DOTMA, DOTA-pNB, DOTP, DOTMP, DOTEP, DOTMPE, F-DOTPME, DOTPP, DOTBzP, DOTA-monoamide, p-NCS-DOTA, p-NCS-PADOTA, BAT, DO3TMP-Monoamide, p-NCS-TRITA, NOTA, CHX-A"-DTPA,
   soit un chélateur bifonctionnel de type cationique, anionique, neutre ou clivable,
   soit un radionucléide choisi de préférence parmi ⁶⁷Cu, ⁶⁴Cu, ⁹⁰Y, ¹⁰⁹ Pd, ¹¹¹Ag, ¹⁴⁹Pm, ¹⁵³Sm, ¹⁶⁶Ho, ¹⁷⁷Lu, ¹⁸⁶Re, ¹⁸⁸Re, ^{99m}Tc, ⁶⁷Ga, ⁶⁸Ga, ¹¹¹In, ⁹⁰Y, ¹⁷⁷Lu, ¹⁸⁶Re, ¹⁸⁸Re, ¹⁹⁷Au, ¹⁹⁸Au, ¹⁹⁹Au, ¹⁰⁵Rh, ¹⁶⁵Ho, ¹⁶⁶Ho, ¹⁶¹Tb, ¹⁴⁹Pm, ⁴⁴Sc, ⁴⁷Sc, ⁷⁰As, ⁷¹As, ⁷²As, ⁷³As, ⁷⁴As, ⁷⁶As, ⁷⁷As, ²¹²Pb, ²¹²Bi, ²¹³Bi, ²²⁵Ac, ^{117m}Sn, ⁶⁷Ga, ²⁰¹Tl, ¹²³I, ¹³¹I, ¹⁶⁰Gd, ¹⁴⁸Nd, ⁸⁹Sr, ²¹¹At,
- un médicament cytotoxique M choisi parmi un agent chimiothérapeutique ou une toxine, en particulier la monométhyle d'auristatine E ou la monométhyle d'auristatine F,
- un radical - L-M, dans lequel L et M ont la signification précédente.

En particulier, les produits de formule IA1 à IA5 du procédé de préparation selon la description sont attachés à la protéine P sur un au moins de ses ponts disulfure par une réaction de substitution nucléophile, typiquement dans un tampon phosphate, en présence de TCEP.

Dans un mode particulier de réalisation de l'invention, les produits de formule IB2 sont utilisés dans le traitement d'une tumeur notamment le cancer colorectal, l'hépatocarcinome, un cancer du poumon, le cancer du pancréas, le cancer du poumon non à petites cellules, le cancer du poumon à petites cellules, le cancer colorectal, le cancer colorectal K-RAS muté, un cancer du sein, le cancer du sein triple-négatif, le cancer du sein métastatique, le cancer du foie, le cancer tête et cou, la maladie de Castleman, le cancer de la thyroïde, le médulloblastome, le glioblastome multiforme, le gliome, le sarcome, l'astrocytome anaplastique, le cancer du rein, le cancer de l'estomac, l'ascite maligne, le cancer de la prostate (métastatique ou non), les tumeurs solides, la leucémie aiguë myéloïde, la leucémie lymphocytaire chronique, le mélanome, le myélome, le myélome multiple, les lymphomes, le lymphome du manteau, le lymphome hodgkinien, le lymphome non-hodgkinien, le lymphome folliculaire.

Dans un autre mode particulier de réalisation de l'invention, les produits de formule IB2 sont utilisés dans le traitement d'une maladie inflammatoire ou auto-immune notamment le rejet de greffe, la polyarthrite rhumatoïde, la maladie de Crohn, le psoriasis, la sclérose en plaques, les syndromes périodiques associés à la cryopyrine, le purpura thrombopénique.

La description concerne également un procédé de préparation des produits de formule IB tels que définis ci-dessus, caractérisé en ce que l'on fait agir un produit de formule IA 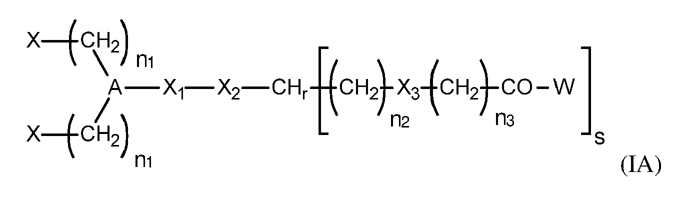 formule (IA) dans laquelle
X est Br ou I, ou un groupe 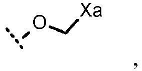 ou un groupe 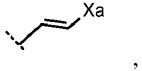 lequel Xₐ est un halogène ou un nucléofuge.
A représente ou bien un radical aryle ou cycloalkyle, lesdits radicaux aryles ou cycloalkyles étant carbocycliques ou hétérocycliques,
ou bien A représente un groupe 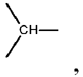 un groupe 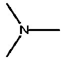 ou un groupe 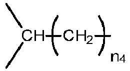
X₁ est un C=O ou un groupe NH ou une simple liaison
X₂ est un groupe NH ou un C=O une simple liaison
X₃ est un oxygène ou une simple liaison
s est égal à 1, 2 ou 3
r est égal à 0, 1 ou 2
   étant entendu que la somme r+s est égale à 3
n₁ est un entier égal à 0 ou 1 ; n₂ est un entier égal à 1, 2 ou 3 et n₃ est un entier égal à 1, 2 ou 3,
n₄ est un entier égal à 1, 2, 3 ou 4,
W représente
   - un radical - L qui représente un corps de linker comportant une fonction réactive terminale,
   - un groupe fluorophore - FL utilisé en diagnostique ou analyse, impliquant une détection de fluorescence choisi de préférence parmi la rhodamine ou l'un de ses dérivés, de préférence la rhodamine B, l'isocyanate de fluorescéine (FITC), une Cy Dye choisie de préférence parmi Cy5, Cy5.5, Cy7, une Alexa fluor Dye choisie de préférence parmi Alexa Fluor 647, 700 ou 750, le Texas Red, le Nile Blue A, l'allophycocyanin (APC) et ses conjugués avec d'autres fluorochromes notamment ceux précédemment cités, la phycoerythrin (PE) et ses conjugués avec d'autres fluorochromes notamment ceux précédemment cités,
   - un radical R* radioactif qui contient de préférence,
      soit un ligand bifonctionnel, éventuellement bimodal, choisi de préférence parmi les dérivés de DOTA, DTPA, C-DOTA, NODAGA, NETA, C-NETA, DEPA, C-DEPA, TETA, TE2A, HYNIC, DAT, MAMA ;
      soit un ligand bifonctionnel choisi de préférence parmi l'un des chélateurs acycliques de la liste constituée par EDTA, CyEDTA, EDTMP, DTPMP, DTPA, CyDTPA, Cy₂DTPA, DTPA-MA, DTPA-BA, BOPA ;
      soit un ligand bifonctionnel choisi de préférence parmi l'un des chélateurs macrocycliques de la liste constituée par DOTA, TRITA, TETA, DOTA-MA, DO3A-HP, DOTMA, DOTA-pNB, DOTP, DOTMP, DOTEP, DOTMPE, F-DOTPME, DOTPP, DOTBzP, DOTA-monoamide, p-NCS-DOTA, p-NCS-PADOTA, BAT, DO3TMP-Monoamide, p-NCS-TRITA, NOTA, CHX-A"-DTPA ;
      soit un chélateur bifonctionnel de type cationique, anionique, neutre ou clivable ;
      soit un radionucléide choisi de préférence parmi ⁶⁷Cu, ⁶⁴Cu, ⁹⁰Y, ¹⁰⁹Pd, ¹¹¹Ag, ¹⁴⁹Pm, ¹⁵³Sm, ¹⁶⁶Ho, ¹⁷⁷Lu, ¹⁸⁶Re, ¹⁸⁸Re, ^{99m}Tc, ⁶⁷Ga, ⁶⁸Ga, ¹¹¹In, ⁹⁰Y, ¹⁷⁷Lu, ¹⁸⁶Re, ¹⁸⁸Re, ¹⁹⁷Au, ¹⁹⁸Au, ¹⁹⁹Au, ¹⁰⁵Rh, ¹⁶⁵Ho, ¹⁶⁶Ho, ¹⁶¹Tb, ¹⁴⁹Pm, ⁴⁴Sc, ⁴⁷Sc, ⁷⁰As, ⁷¹As, ⁷²As, ⁷³As, ⁷⁴As, ⁷⁶As, ⁷⁷As, ²¹²Pb, ²¹²Bi, ²¹³Bi, ²²⁵Ac, ^{117m}Sn, ⁶⁷Ga, ²⁰¹Tl, ¹²³I, ¹³¹I, ¹⁶⁰Gd, ¹⁴⁸Nd, ⁸⁹Sr, ²¹¹At,
   - un médicament cytotoxique M choisi parmi un agent chimiothérapeutique ou une toxine, en particulier la monométhyle d'auristatine E ou la monométhyle d'auristatine F,
   - un radical - L-M dans lequel, L et M ont la signification précédente,
   en particulier le groupe 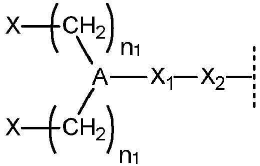 est choisi parmi les radicaux : 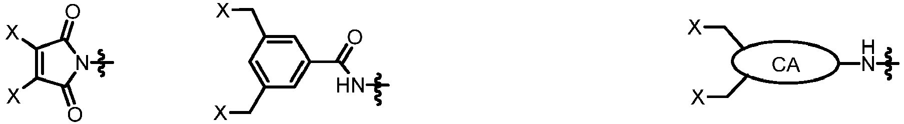 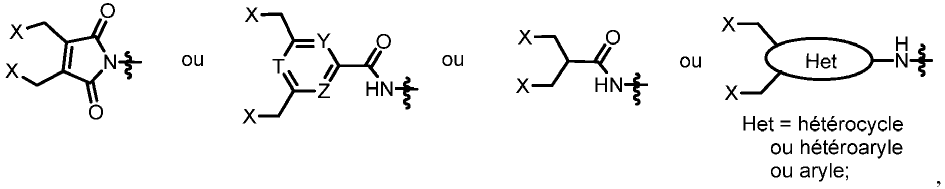 ainsi que les dérivés de la ou des fonctions acides carboxyliques tels que les sels, les esters ou les amides, de préférence l'amide formée avec le butanimide,
   avec un produit de formule P, dans laquelle P est une protéine comprenant au moins un pont disulfure, pour obtenir un produit de formule (IB) : 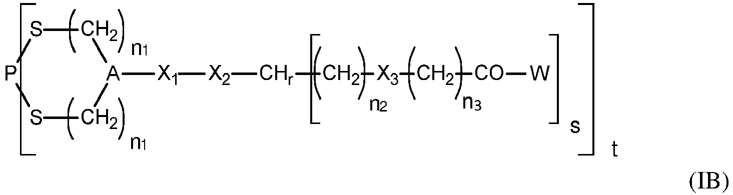
dans laquelle t représente un entier de 1 à 15, de préférence de 1 à 6 et 13.

### DESCRIPTION DES FIGURES

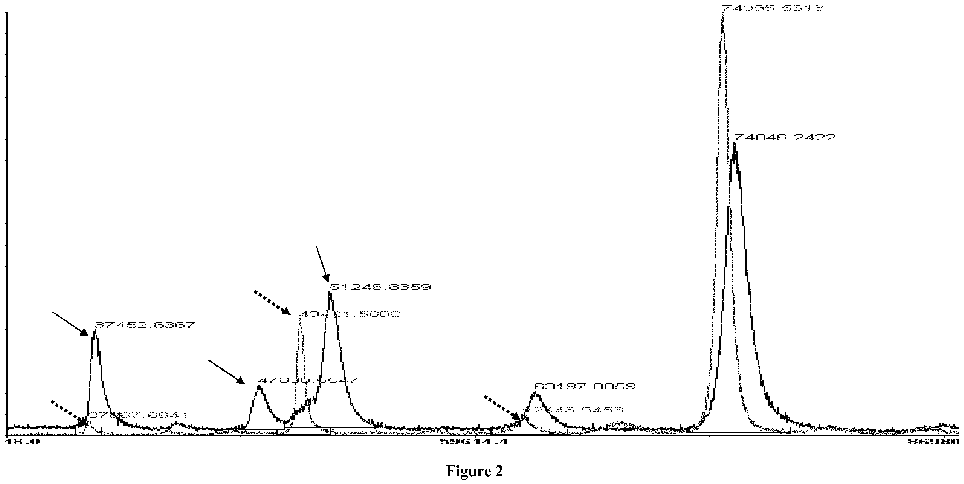

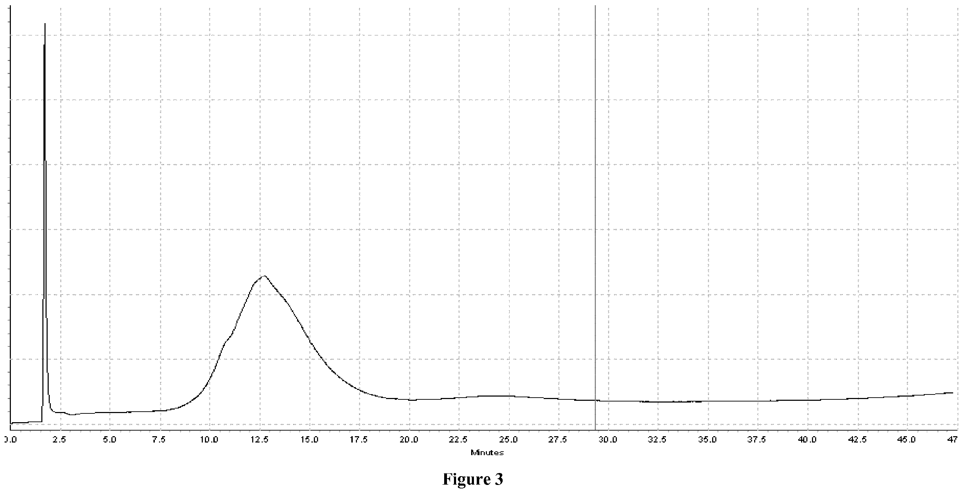

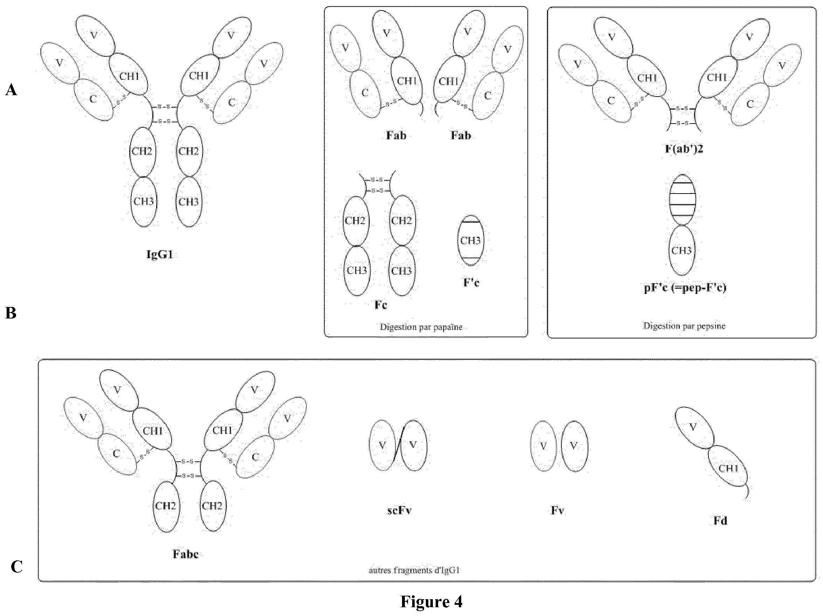

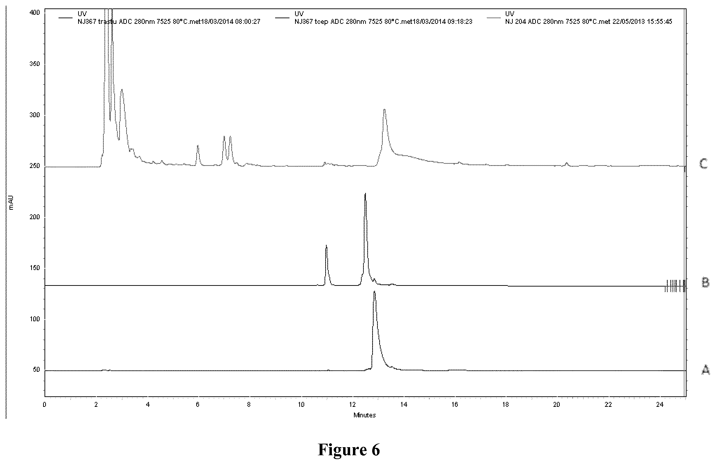

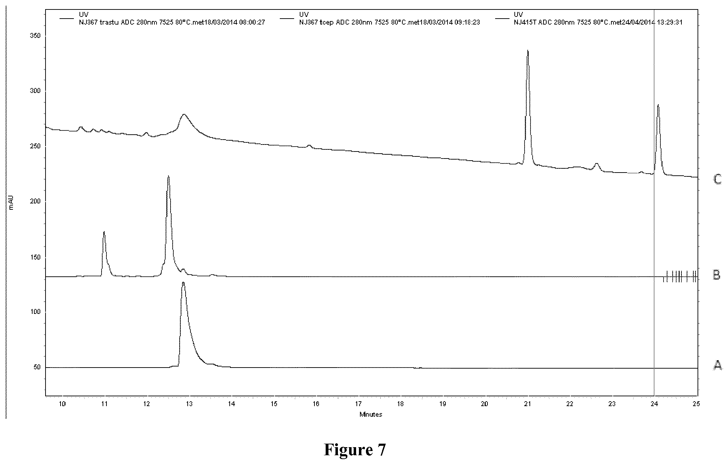

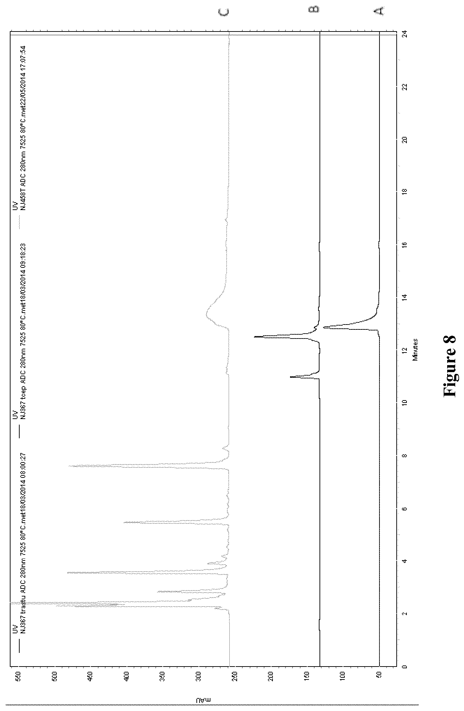

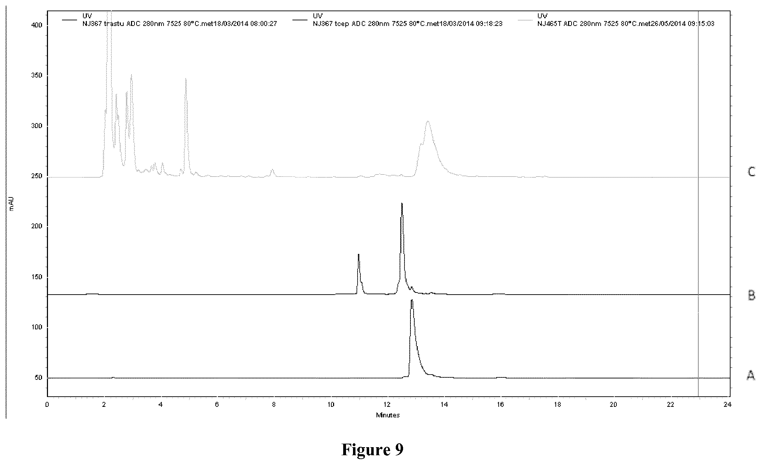

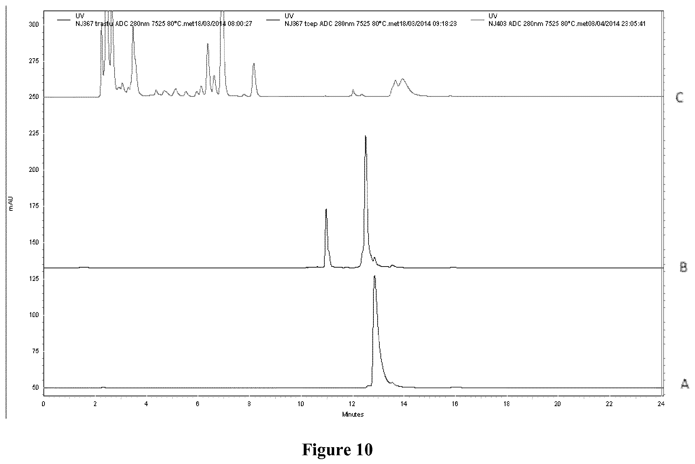

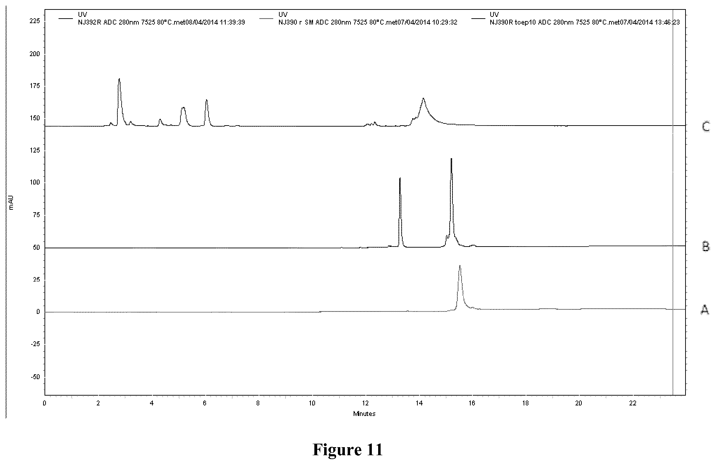

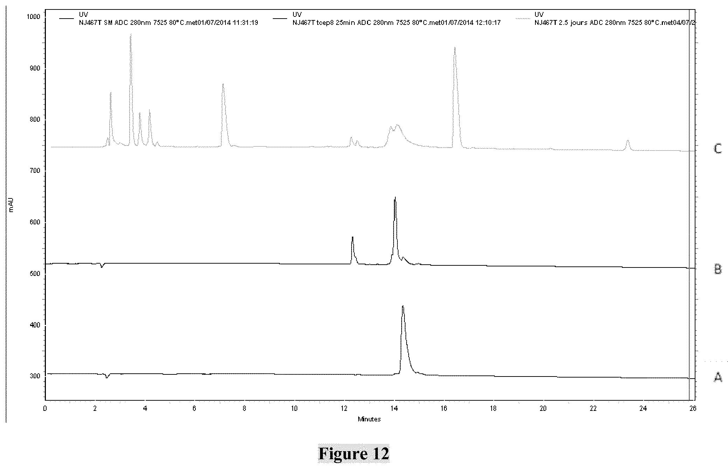

**La** **figure 1** présente la structure générale d'un conjugué anticorps-médicament.
   Les sites classiques de modification sont les suivants :
   - 4 lysines
      Moyenne de 4 résidus modifiés parmi les 8 résidus accessibles pour modification parmi 80, par conséquent nombreuses espèces de D.A.R.
   - 4 ponts disulfures
   Après réduction au TCEP (*tris*(2-carboxyethyl)phosphine), 8 résidus cystéines accessibles, par conséquent nombreuses espèces de D.A.R de 0 à 8. D.A.R = Ratio drug-anticorps (ou drug-to-antibody ratio).
   Le D.A.R moyen est d'environ 4 avec des modifications classiques.
**La** **figure 2** présente l'analyse par spectrométrie de masse MALDI-TOF du trastuzumab (pic à 74095 et pics indiqués par les flèches en pointillés) et du trastuzumab greffé reconstruit par le linker (espaceur) acide 6-(3,4-dibromomaleimido)hexanoïque (pic à 74846 et pics indiqués par les flèches pleines).
   Cette analyse permet de confirmer que le mélange réactionnel (pic à 74095) contient à 89 % l'espèce résultant du greffage de 4 linkers d'acide 6-(3,4-dibromomaleimido)hexanoïque sur l'anticorps, la moyenne de greffage est 4.0 : 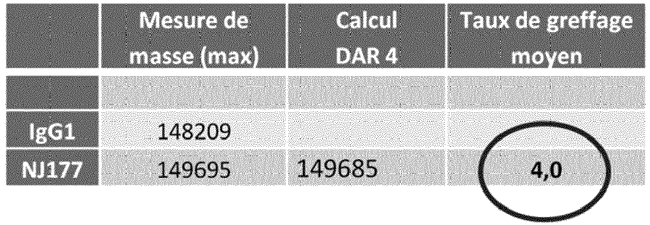 Calcul de la répartition des différentes espèces par déconvolution du pic principal (pic à 74846) : 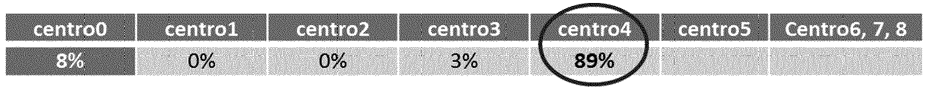
**La** **figure 3** présente le chromatogramme HIC-HPLC de l'anticorps trastuzumab **24** purifié et reconstruit après réduction par le TCEP puis ajout du linker 6-(3,4-dibromomaleimido)hexanamide-Val-Cit-PAB-OH.
   Une petite quantité de DAR=0 demeure (confirmation des résultats MALDI-TOF) et DAR=4 est l'espèce principale.
**Les** **figures 4A à 4C** présentent la nomenclature des différentes fractions d'anticorps.
   La figure 4A présente les fragments d'Ig1 obtenus après digestion à la papaïne (Fc et F'c) ou la pepsine (Fc' = pepF'c).
   La figure 4B présente les fragments Fabc, Fd, Fv et scFv.
   La figure 4C présente la structure des anticorps chimériques et humanisés.
**La** **figure 5** présente le chromatogramme RP-HPLC de l'anticorps trastuzumab-(maldiBr-linker**7**)₄ **24** obtenu dans l'exemple 5.2.
   A : trastuzumab.
   B : anticorps réduit par le TCEP : les chaines lourdes et légères de trastuzumab sont séparées due à la nature dénaturante de l'analyse RP-HPLC.
   C : trastuzumab **24** reconstruit (mélange réactionnel brut) : un seul pic est observé, la preuve que les ponts entre les chaînes ont été reconstruits par le linker **7** acide hexanoïque 6-(3,4-dibromomaleimido).
   Les pics où les temps de rétention sont inférieurs à 10 minutes représentent les résidus linker.
**La** **figure 6** présente l'analyse RP-HPLC du trastuzumab-(maldiBr-linker**8**)₄ **25** obtenu dans l'exemple 5.3.
   A : trastuzumab natif.
   B : trastuzumab réduit par TCEP.
   C : trastuzumab **25** reconstruit par le linker **8** 6-(3,4-dibromomaleimido)hexanamide-Val-Cit-PAB-OH.
**La** **figure 7** présente l'analyse RP-HPLC du trastuzumab-(maldiSPh-linker**26**)₄ **27** obtenu dans l'exemple 5.4.
   A : trastuzumab natif.
   B : trastuzumab réduit par le TCEP.
   C : trastuzumab **27** reconstruit par le linker **26** 6-(3,4-dithiophenylmaleimido)hexanamide-Val-Cit-PAB-OH.
**La** **figure 8** présente l'analyse RP-HPLC du trastuzumab-(PydiMediBr-linker**20**)₄ **28** obtenu dans l'exemple 5.5.
   A : trastuzumab natif.
   B : trastuzumab réduit par le TCEP.
   C : trastuzumab **28** par le linker **20** acide hexanoique 6-(2,6-bis(bromomethyl)isonicotinamido).
**La** **figure 9** présente l'analyse RP-HPLC du trastuzumab-(PydiMediBr-linker**22**)₄ **29** obtenu dans l'exemple 5.6.
   A : trastuzumab natif.
   B : trastuzumab réduit par le TCEP.
   C : trastuzumab **29** par le linker acide **22** 6-(2,6-bis(bromomethyl)isonicotinamido)hexanamide-Val-Cit-PAB-OH.
**La** **figure 10** présente l'analyse RP-HPLC du trastuzumab-(PhdiMediBr-linker**15**)₄ **30** obtenu dans l'exemple 5.7.
   A : trastuzumab natif.
   B : trastuzumab réduit par le TCEP.
   C : Trastuzamab **30** partiellement reconstruit par le linker **15** acide 6-(2,6-bis(bromomethyl)benzamido)hexanamide-Val-Cit-PAB-OH.
**La** **figure 11** présente l'analyse RP-HPLC du rituximab-(maldiBr-linker**7**)₄ **31** obtenu dans l'exemple 5.8.
   A : rituximab natif.
   B : rituximab réduit par le TCEP.
   C : rituximab **31** reconstruit par le linker **7** acide hexanoique 6-(3,4-dibromomaleimido).
**La** **figure 12** présente l'analyse RP-HPLC du trastuzumab-(maldiBr-linker**9-MMAE**)₄ **32** obtenu dans l'exemple 5.9.
   A : trastuzumab natif.
   B : trastuzumab réduit par le TCEP.
   C : trastuzumab **32** reconstruit par le linker **9-MMAE** 6-(3,4-dibromomaleimido)hexanamide-Val-Cit-PAB-MMAE.

### EXEMPLES :

### Exemple 1 : Groupement valine-citrulline- alcool para-aminobenzyle

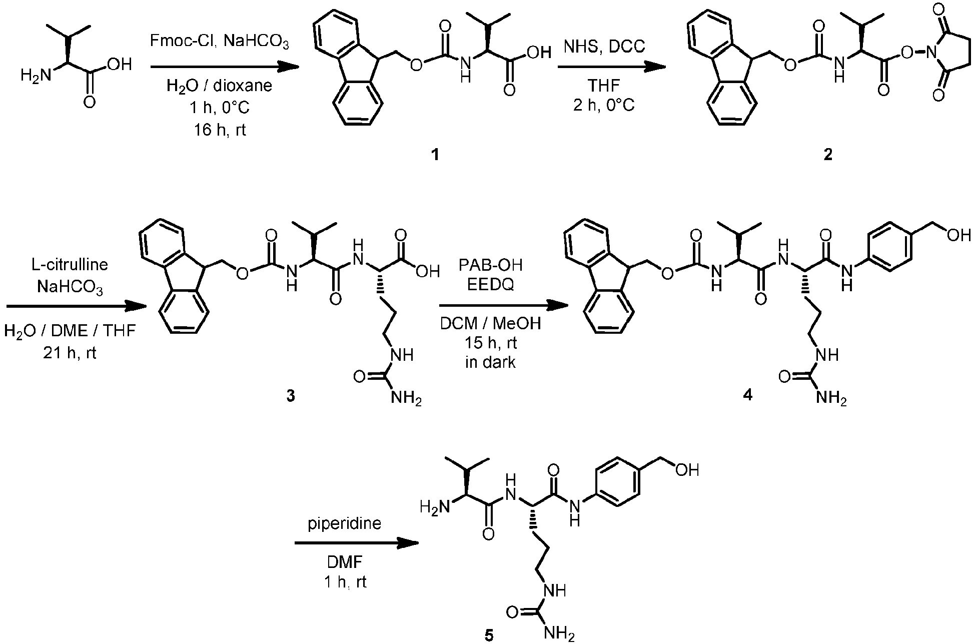

### 1.1. Synthèse de Fmoc-Val-OH (1)

La valine (1,09 g, 9,31 mmol, 1éq) est dissoute dans 20 mL d'eau puis 20 mL de dioxane et NaHCO₃ (1,56 g, 18,62 mmol, 2 éq) sont ajoutés. Le milieu est refroidi à 0°C puis le Fmoc-Cl (2,65 g, 10,24 mmol, 1,1 éq) est inséré et la réaction est laissée 1h à 0°C puis agitée la nuit à température ambiante. Le lendemain, le milieu réactionnel est acidifié jusqu'à pH=2 avec une solution de HCl 1 M puis extrait avec AcOEt (3 x 60 mL). Après évaporation sous vide, l'huile jaune obtenue est purifiée par chromatographie flash de 0 à 40% d'AcOEt dans le cyclohexane. Un solide blanc est obtenu (2,23 g, 71%).

¹H NMR (300 MHz, CDCl₃) : δ(ppm) 7,78-7,29 (m, 8H ; Ar Fmoc), 5,25 (d, *J*= 9,0 Hz, 1H ; CON*H* Val), 4,43 (d, *J*= 7,0 Hz, 2H ; C*H*₂ Fmoc), 4,37 (dd, *J*= 4,5 Hz, *J*= 9,0 Hz, 1H ; *H*_{α} Val), 4,24 (t, *J*= 7,0 Hz, 1H ; CH Fmoc), 2,29-2,23 (m, 1H ; *H*_{β} Val), 1,02, 0,96 (2d, *J*= 7,0 Hz, 6H ; C*H*₃ Val)

¹³C NMR (75 MHz, CDCl₃) : δ(ppm) 176,50 (1C, COOH), 156,48 (1C, C=O Fmoc), 143,85, 141,47 (4C, C-Ar Fmoc), 127,89, 127,23, 125,21, 120,16 (8C, CH-Ar Fmoc), 67,27 (1C, CH₂ Fmoc), 58,93 (1C, C_{α} Val), 47,32 (1C, CH Fmoc), 31,15 (1C, C_{β} Val), 19,17, 17,59 (2C, CH₃ Val)

### 1.2. Synthèse de Fmoc-Val-OSu (2)

Le Fmoc-Val-OH (735 mg, 2,17 mmol, 1 éq) et la N-hydroxysuccinimide (375 mg, 3,26 mmol, 1,5 éq) sont dissous dans 5,5 mL de THF. La DCC (448 mg, 2,17 mmol, 1 éq) est ajoutée à 0°C et la réaction est agitée pendant 2h à cette température. Le milieu est ensuite filtré sur fritté et le solide est lavé- au THF (3 x 3 mL). La phase organique est séchée sur MgSO₄ et évaporée sous vide puis purifiée par chromatographie flash de 0 à 40% d'AcOEt dans le cyclohexane pour donner une gomme incolore (865 mg, 91%).

HRMS : m/z calculé : 437,10671 [M + H]+, trouvé: 437,17074.

¹H NMR (300 MHz, CDCl₃) : δ(ppm) 7,78-7,29 (m, 8H ; Ar Fmoc), 5,28 (d, *J*= 9,0 Hz, 1H ; CON*H* Val), 4,69 (dd, *J*= 5,0 Hz, *J*= 9,0 Hz, 1H ; *H*_{α} Val), 4,45 (d, *J*= 7,0 Hz, 2H ; C*H*₂ Fmoc), 4,24 (t, *J*= 7,0 Hz, 1H ; CH Fmoc), 2,84 (s, 4H ; NCOC*H*₂), 2,40-2,29 (m, 1H ; *H*_{β} Val), 1,07, 1,05 (2d, *J*= 7,0 Hz, 6H ; C*H*₃ Val)

¹³C NMR (75 MHz, CDCl₃) : δ(ppm) 168,72, 167,87 (3C, C=O Su, C=O Val), 155,97 (1C, C=O Fmoc), 143,94, 143,72, 141,42 (4C, C-Ar Fmoc), 127,85, 127,23, 125,17, 120,10 (8C, CH-Ar Fmoc), 67,35 (1C, CH₂ Fmoc), 57,60 (1C, C_{α} Val), 47,25 (1C, CH Fmoc), 31,75 (1C, C_{β} Val), 25,69 (2C, C*H*₂C(O)NO), 18,82, 17,41 (2C, CH₃ Val)

### 1.3. Synthèse de Fmoc-Val-Cit-OH (3)

La L-citrulline (84 mg, 0,481 mmol, 1,05 éq) et NaHCO₃ (40 mg, 0,481 mmol, 1,05 éq) sont dissous dans 1,2 mL d'eau et une solution de Fmoc-Val-OSu (200 mg, 0,458 mmol, 1 éq) dans 1,2 mL de DME y est introduite. Pour améliorer la solubilité, 1,2 mL de THF sont ajoutés et le milieu est agité pendant 21h. 2,3 mL d'acide citrique 15% sont ajoutés et le milieu précipite ; il est extrait avec un mélange 10% iPrOH/AcOEt (3 x 3 mL). Les phases organiques réunies sont lavées avec une solution de NaCl (2 x 4,5 mL), séchées sur MgSO₄ puis évaporées. Quand le solide blanc obtenu est bien sec, il est lavé avec de l'éther pour donner un solide blanc électrostatique (140 mg, 62%).

HRMS: m/z calculé: 497,26142 [M + H]+, trouvé: 497,23945.

¹H NMR (300 MHz, DMSO-*d₆*) : δ(ppm) 12,50 (br s, 1H ; COOH), 8,17 (d, *J*= 7,5 Hz, 1H ; CON*H* Cit), 7,88-7,27 (m, 9H ; Ar Fmoc, CONH Val), 5,91 (t, *J*= 5,5 Hz, 1H ; NH Cit), 5,36 (s, 2H ; N*H*₂ Cit), 4,25-4,07 (m, 4H ; C*H*₂ Fmoc, CH Fmoc, *H*_{α} Cit), 3,89 (dd, *J*= 7,0 Hz, *J*= 9,0 Hz, 1H ; *H*_{α} Val), 2,94-2,88 (m, 2H ; NC*H*₂ Cit), 1,97-1,88 (m, 1H ; *H*_{β} Val), 1,70-1,31 (m, 4H ; C*H*₂ Cit), 0,86, 0,82 (2d, *J*= 7,0 Hz, 6H ; C*H*₃ Val)

¹³C NMR (75 MHz, DMSO-*d₆*): δ(ppm) 173,45, 171,31 (2C, COOH, C=O Val), 158,76 (1C, NH₂CO Cit), 156,07 (1C, C=O Fmoc), 143,95, 143,80, 140,72 (4C, C-Ar Fmoc), 127,67, 127,09, 125,43, 120,11 (8C, CH-Ar Fmoc), 65,69 (1C, CH₂ Fmoc), 59,83 (1C, C_{α} Val), 51,90 (1C, C_{α} Cit), 46,69 (1C, CH Fmoc), 38,76 (1C, NC*H*₂ Cit), 30,56 (1C, C_{β} Val), 28,38, 26,71 (2C, CH₂ Cit), 19,19, 18,24 (2C, CH₃ Val)

### 1.4. Synthèse de Fmoc-Val-Cit-PAB-OH (4)

Fmoc-Val-Cit-OH (518 mg, 1,04 mmol, 1 éq) et le p-aminobenzyl alcohol (256 mg, 2,08 mmol, 2 éq) sont dissous dans 18 mL de DCM/MeOH 2/1 et l'EEDQ (514 mg, 2,08 mmol, 2 éq) est ajouté. Le milieu est agité pendant 15h dans le noir à température ambiante. Les solvants sont évaporés, et le solide jaune obtenu est trituré avec de l'éther. La suspension est passée aux ultrasons pendant 5 min et laissée reposer 30 min. Elle est ensuite filtrée et le solide est rincé plusieurs fois à l'éther pour donner une poudre jaune (559 mg, 89%).

HRMS: m/z calculé: 601,85499 [M + H]+, trouvé: 602,29758.

¹H NMR (300 MHz, DMSO-*d₆*) : δ(ppm) 9,97 (s, 1H ; NH PAB), 8,10 (d, *J*= 7,0 Hz, 1H ; CONH Cit), 7,87-7,18 (m, 13H ; Ar Fmoc, Ar PAB, CONH Val), 5,95 (br t, *J*= 5,5 Hz, 1H ; *NH* Cit), 5,40 (s, 2H ; N*H*₂ Cit), 5,09 (t, *J*= 5,5 Hz, 1H ; O*H* PAB), 5,39 (d, *J*= 5,5 Hz, 1H ; C*H*₂ PAB), 4,27-4,19 (m, 4H ; C*H*₂ Fmoc, C*H* Fmoc, *H*_{α} Cit), 3,89 (dd, *J*= 8,0 Hz, *J*= 8,0 Hz, 1H ; *H*_{α} Val), 3,03-2,84 (m, 2H ; NC*H*₂ Cit), 1,98-1,92 (m, 1H ; *H*_{β} Val), 1,69-1,27 (m, 4H ; C*H*₂ Cit), 0,84, 0,82 (2d, *J*= 7,0 Hz, 6H ; C*H*₃ Val)

¹³C NMR (75 MHz, DMSO-*d₆*) : δ(ppm) 171,30, 170,43 (2C, C=O Cit, C=O Val), 158,94 (1C, NH₂CO Cit), 156,17 (1C, C=O Fmoc), 143,94, 143,81, 140,75 (4C, C-Ar Fmoc), 137,55, 137,48 (2C, C-Ar PAB), 127,70, 127,13, 125,40, 120,14 (8C, CH-Ar Fmoc), 126,98, 118,91 (4C, CH-Ar PAB), 65,73 (1C, CH₂ Fmoc), 62,63 (1C, CH₂ PAB), 60,14 (1C, C_{α} Val), 53,11 (1C, C_{α} Cit), 46,72 (1C, CH Fmoc), 38,96 (1C, NC*H*₂ Cit), 30,48 (1C, C_{β} Val), 29,57, 26,81 (2C, CH₂ Cit), 19,26, 18,31 (2C, CH₃ Val)

### 1.5. Synthèse de H-Val-Cit-PAB-OH (5)

Fmoc-Val-Cit-PAB-OH (298 mg, 0,495 mmol, 1 éq) est dissous dans 4 mL de DMF et 1 mL de pipéridine est ajouté. Après 1h de réaction le milieu est évaporé. Le solide visqueux obtenu est trituré avec du DCM pour donner un solide beige après filtration (189 mg, 100%).

HRMS: m/z calculé: 601,85499 [M + H]+, trouvé: 602,29758.

¹H NMR (300 MHz, DMSO-*d₆*) : δ(ppm) 10,04 (s, 1H ; NH PAB), 8,16 (d, *J*= 7,5 Hz, 1H ; CONH Cit), 7,52, 7,22 (2d, *J*= 8,5 Hz, 4H ; Ar PAB), 5,97 (br t, *J*= 5,5 Hz, 1H ; NH Cit), 5,40 (s, 2H ; N*H*₂ Cit), 5,10 (br t, *J*= 5,5 Hz, 1H ; OH PAB), 4,46-4,40 (m, 3H ; C*H*₂ PAB, *H*_{α} Cit), 3,52-3,18 (N*H*₂ Val, eau), 3,05-2,87 (m, 3H ; *H*_{α} Val, NC*H*₂ Cit), 1,95-1,89 (m, 1H ; *H*_{β} Val), 1,71-1,28 (m, 4H ; C*H*₂ Cit), 0,87, 0,77 (2d, *J*= 7,0 Hz, 6H ; C*H*₃ Val)

¹³C NMR (75 MHz, DMSO-*d₆*) : δ(ppm) 170,48 (2C, C=O Cit, C=O Val), 158,86 (1C, NH₂CO Cit), 137,52, 137,43 (2C, C-Ar PAB), 126,94, 118,95 (4C, CH-Ar PAB), 62,59 (1C, CH₂ PAB), 59,50 (1C, C_{α} Val), 52,53 (1C, C_{α} Cit), 38,08 (1C, NC*H*₂ Cit), 31,25 (1C, C_{β} Val), 30,10, 26,70 (2C, CH₂ Cit), 19,46, 16,96 (2C, CH₃ Val)

### Exemple 2 : Dérivé de maléimide comme tête de bioconjugaison (non couvert par la présente invention)

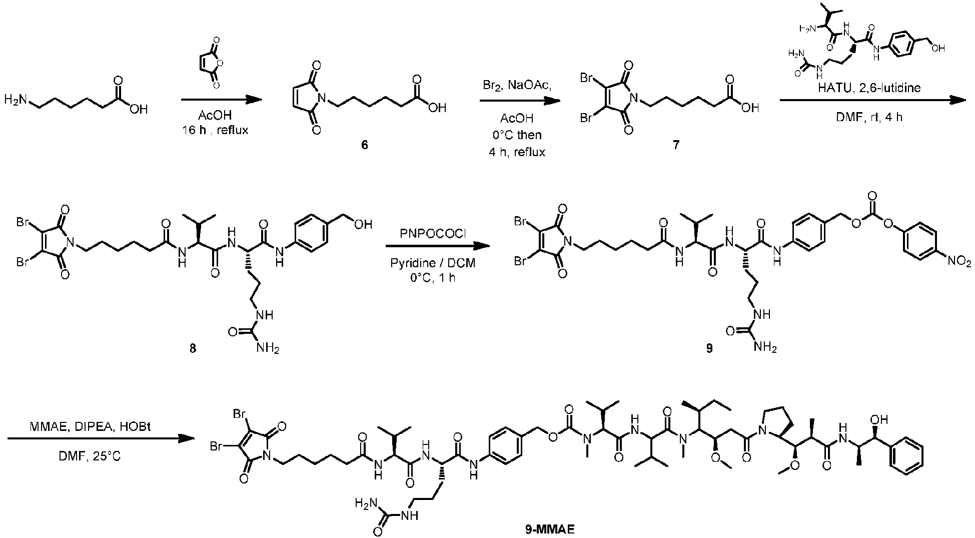

### 2.1. Synthèse de l'acide 6-maleimidohexanoique (6)

L'anhydride maléique (1,50 g, 15,25 mmol, 1éq) et l'acide 6-aminocaproïque (2,00 g, 15,25 mmol, 1 éq) sont agités sous argon dans 15 mL d'acide acétique. Le milieu réactionnel est chauffé à reflux et agité pendant une nuit. Le lendemain, le mélange est devenu orange. L'acide acétique est coévaporé trois fois avec 75 mL de toluène et une huile visqueuse orangée est obtenue ; elle est reprise dans 150 mL de DCM puis lavée avec 150 mL d'eau. La phase aqueuse est réextraite (2 x 150 mL) et les phases organiques réunies sont séchées sur MgSO₄ puis évaporées sous vide pour donner un solide orangé (2,60 g, 81%).

HRMS: m/z calculé: 216,95765 [M + H]⁺, trouvé: 217,09071.

¹H NMR (300 MHz, CDCl₃) : δ(ppm) 6,69 (s, 2H ; C*H*=C*H*), 3,51 (t, *J*= 7,0 Hz, 2H ; NC*H*₂), 2,34 (t, *J*= 7,5 Hz, 2H ; C*H*₂COOH), 1,70-1,55 (m, 4H ; NCH₂C*H*₂CH₂C*H*₂), 1,38-1,28 (m, 2H ; NCH₂CH₂C*H*₂)

¹³C NMR (75 MHz, CDCl₃) : δ(ppm) 179,53 (1C, COOH), 171,00 (2C, C=O MC), 134,21 (2C, CH=CH), 37,71 (1C, NCH₂), 33,86 (1C, C*H*₂COOH), 28,30 (1C, NCH₂C*H*₂), 26,24 (1C, NCH₂CH₂C*H*₂), 24,21 (1C, C*H*₂CH₂COOH)

### 2.2. Synthèse de l'acide 6-(3,4-dibromomaleimido)hexanoique (7)

L'acide maleimidohexanoique (200 mg, 0,947 mmol, 1 éq) et l'acétate de sodium (117 mg, 1,421 mmol, 1,5 éq) sont introduits dans un tube mis sous argon et 2,9 mL d'acide acétique sont ajoutés. Du dibrome (0,07 mL, 1,421 mmol, 1,5 éq) est introduit à 0°C, le tube est scellé puis chauffé à reflux pendant 4h. Le milieu est ensuite laissé revenir à température ambiante et 16 mL d'eau glacée sont ajoutés. La phase aqueuse est extraite à l'AcOEt (3 x 20 mL) et les phases organiques réunies sont lavées avec 40 mL d'une solution de thiosulfate de sodium, séchées sur MgSO₄, et coévaporées au toluène. Le résidu marron obtenu est purifié par chromatographie flash de 0 à 40% d'AcOEt dans le cyclohexane pour donner un solide jaune (237 mg, 68%).

HRMS: m/z calculé: 368,47706 [M + H]+, trouvé: 367,91255

¹H NMR (300 MHz, CDCl₃) : δ(ppm) 3,62 (t, *J*= 7,0 Hz, 2H ; NC*H*₂), 2,36 (t, *J*= 7,5 Hz, 2H ; C*H*₂COOH), 1,72-1,59 (m, 4H ; NCH₂C*H*₂CH₂C*H*₂), 1,42-1,31 (m, 2H ; NCH₂CH₂C*H*₂)

¹³C NMR (75 MHz, CDCl₃) : δ(ppm) 178,18 (1C, COOH), 164,10 (2C, C=O MC), 129,50 (2C, C=C), 39,63 (1C, NCH₂), 33,60 (1C, C*H*₂COOH), 28,25 (1C, NCH₂C*H*₂), 26,16 (1C, NCH₂CH₂C*H*₂), 24,17 (1C, C*H*₂CH₂COOH)

### 2.3. Synthèse de 6-(3,4-dibromomaleimido)hexanamide-Val-Cit-PAB-OH (8)

L'acide 6-(3,4-dibromomaleimido)hexanoïque (178 mg, 0,482 mmol, 1 éq) et du HATU (238 mg, 0,627 mmol, 1,3 éq) sont dissous dans 2,2 mL de DMF anhydre puis la 2,6-lutidine (73 µL, 0,627 mmol, 1,3 éq) est ajoutée. Le milieu est agité pendant 1h puis le dipeptide (183 mg, 0,482 mmol, 1 éq) est ajoutée et la réaction est laissée la nuit sous agitation. 1,6 mL de HCl 1 M sont ensuite ajoutés et la phase aqueuse est extraite deux fois avec de l'AcOEt. Les phases organiques réunies sont lavées trois fois avec une solution de NaCl saturée, puis évaporées. Le résidu obtenu est purifié par chromatographie sur colonne de gel de silice avec un gradient d'éluant DCM/MeOH de 15/1 à 9/1 pour donner un solide jaune (84 mg, 24%).

HRMS: m/z calculé: 729,15941 [M + H]+, trouvé: 729,12412.

¹H NMR (300 MHz, DMSO-*d₆*) : δ(ppm) 9,91 (s, 1H ; NH PAB), 8,08 (d, *J*= 8,0 Hz, 1H ; CONH Cit), 7,83 (d, *J*= 8,5 Hz, 1H ; CON*H* Val), 7,54, 7,22 (2d, *J*= 8,5 Hz, 4H ; Ar PAB), 5,98 (br t, *J*= 5,5 Hz, 1H ; *NH* Cit), 5,42 (s, 2H ; N*H*₂ Cit), 5,15-5,07 (m, 1H ; *OH* PAB), 4,42-4,33 (m, 3H ; C*H*₂ PAB, *H*_{α} Cit), 4,19 (dd, *J*= 7,0 Hz, *J*= 8,5 Hz, 1H ; *H*_{α} Val), 3,46-3,27 (NC*H*₂ MC, eau), 3,05-2,89 (m, 2H ; NC*H*₂ Cit), 2,23-2,08 (m, 2H ; C*H*₂CO MC), 2,01-1,91 (m, 1H ; *H*_{β} Val), 1,73-1,16 (m, 10H ; C*H*₂ Cit, NCH₂C*H*₂C*H*₂C*H*₂), 0,85, 0,81 (2d, *J*= 7,0 Hz, 6H ; C*H*₃ Val)

¹³C NMR (75 MHz, DMSO-*d₆*) : δ(ppm) 172,33, 171,28, 170,39, 163,13 (5C, C=O Cit, C=O Val, C=O MC), 158,92 (1C, NH₂CO Cit), 137,54, 137,42 (2C, C-Ar PAB), 132,30 (2C, C=C), 126,94, 118,86 (4C, CH-Ar PAB), 62,60 (1C, CH₂ PAB), 57,61 (1C, C_{α} Val), 53,08 (1C, C_{α} Cit), 38,96 (1C, NCH₂ MC), 38,68 (1C, NC*H*₂ Cit), 34,94 (1C, C*H*₂CO MC), 30,39 (1C, C_{β} Val), 29,39, 27,55, 26,78, 25,70, 24,91 (5C, NCH₂C*H*₂C*H*₂C*H*₂, C*H*₂ Cit), 19,26, 18,18 (2C, CH₃ Val)

### 2.4. Synthèse de 6-(3,4-dibromomaleimido)hexanamide-Val-Cit-PAB-OCOOPNP (9)

Le 6-(3,4-dibromomaleimido)hexanamide-Val-Cit-PAB-OH (42 mg, 57,5 µmol, 1 éq) est dissout dans 0,85 mL de pyridine anhydre. Le PNPOCOCl (35 mg, 172,5 µmol, 3 éq) est dissout dans 0,6 mL de DCM anhydre puis ajouté à la solution du 6-(3,4-dibromomaleimido)hexanamide-Val-Cit-PAB-OH dans la pyridine à 0°C. Au bout d'1h il n'y a plus de produit de départ. La pyridine est coévaporé trois fois au toluène puis le résidu est purifié par chromatographie sur colonne avec un gradient d'éluant DCM/MeOH de 15/1 à 8/2 pour donner un solide orangé (5,9 mg, 11%).

Le produit est utilisé directement pour greffage sur la molécule d'intérêt (c'est-à-dire le médicament, le fluorochrome, etc).

### 2.5. Synthèse de 6-(3,4-dibromomaleimido)hexanamide-Val-Cit-PAB-MMAE (9-MMAE)

Le 6-(3,4-dibromomaleimido)hexanamide-Val-Cit-PAB-OCOOPNP (9,0 mg, 10 µmol, 1 éq) est dissout sous argon à 25°C dans 1 mL de mélange anhydre DMF / pyridine : 4/1. A cette solution sont successivement ajoutés la DIPEA (1,4 µL, 1,2 éq), le HOBt (1,83 mg, 1 éq) et la MMAE (7,2 mg, 10 µmol), 1éq). Le mélange est laissé sous agitation pendant 24 h (suivi HPLC) et le brut réactionnel est purifié par HPLC semi-préparative pour fournir le linker 9-MMAE (4,3 mg, 29%) sous forme de solide jaune, dont la conformité est vérifiée par spectroscopie de masse.

### Exemple 3 : Dérivé de benzène comme tête de bioconjugaison

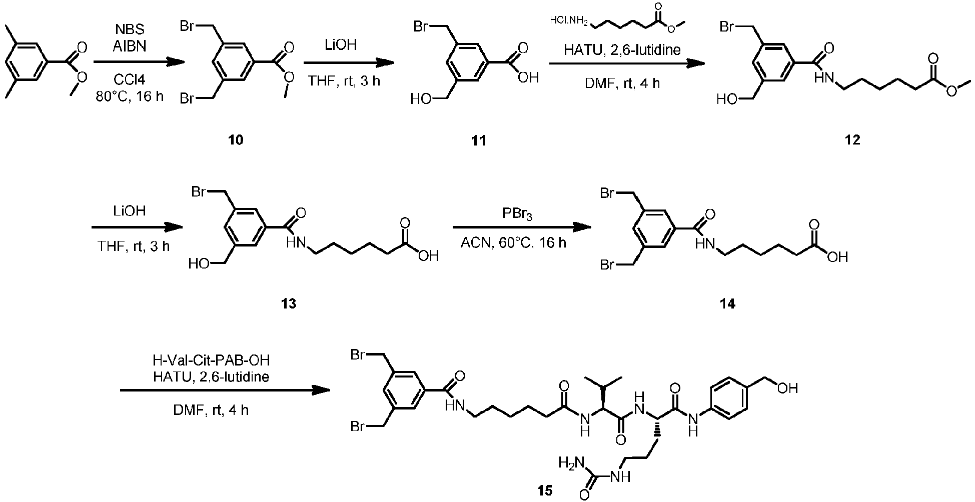

### 3.1. Méthyle 3,5-bis (bromomethyl) benzoate (10)

Le méthyle 3,5-bis (bromomethyl) benzoate (1.00g, 6.09 mmol, 1 eq) est dissout dans du CCl₄ anhydre (25mL) et le mélange est chauffé à reflux. Le NBS (2.38 g, 13.40 mmol, 2.2 eq) est ajouté en cinq portions égales pendant 8h suivi par l'addition de quelques milligrammes de Bz₂O₂. Le mélange est agité toute la nuit et ensuite filtré. Le filtrat est lavé avec de l'eau (50 mL), une solution saturée de NaHCO₃ (25 mL) et de la saumure (30 mL). La couche organique est séchée sur du MgSO₄ et évaporée. Le résidu est dissout dans 25 ml de THF anhydre et du phosphite de diéthyle (1.7 mL, 13.40 mmol, 2.2 eq) et de la DIPEA anhydre (2.3 mL, 13.40 mmol, 2.2 eq) est ajoutée à 0°C. Le mélange est ensuite agité à température ambiante pendant deux jours. Il est évaporé et 50 mL d'eau glacée sont ajoutés et extraits avec de l'éther (3 x 25 mL). Les couches organiques combinées sont lavées avec une solution de 1M HCL (25 mL), de la saumure (25 mL) et sont séchées sur du MgSO₄. L'évaporation du solvant sous pression réduite a donné un résidu qui est purifié par chromatographie flash dans un gradient de cyclohexane à 5% d'ETOAc dans le cyclohexane pour donner **10** sous forme d'un solide blanc (0.83 g, 42%).

¹H NMR (300 MHz, CDCl₃): δ(ppm) 8,00 (d, *J*= 2,0 Hz, 2H; *H*-2, *H*-6), 7,62 (t, *J*= 2,0 Hz, 1H; *H*-4), 4,50 (s, 4H; C*H*₂Br), 3,94 (s, 3H; COC*H*₃).

¹³C NMR (75 MHz, CDCl₃): δ(ppm) 166,07 (1C, COO), 139,09 (2C, C-3, C-5), 134,00 (1C, CH-4), 131,55 (1C, C-1), 130,18 (2C, CH-2, CH-6), 52,57 (1C, OCH₃), 31,99 (2C, C*H*₂Br).

### 3.2. Procédure générale 1 pour la saponification avec l'hydroxyde de lithium :

L'ester (1 mmol) est dissout dans du THF (20 mL) et du LiOH est ajouté (2.5 eq). Le mélange réactionnel est agité à température ambiante jusqu'au terme de la réaction (généralement de 3 h, vérifié par un suivi CCM). Ensuite, 1M de HCl est ajouté jusqu'à ce que le pH de la solution devienne acide (pH=2), et le mélange est extrait avec du EtOAc (3 x 25 mL). Les couches organiques combinées sont séchées sur du MgSO₄. L'évaporation du solvant sous pression réduite permet d'obtenir l'acide correspondant.

### 3.3. Procédure générale 2 pour un couplage peptidique utilisant le HATU :

L'acide (0.2 mmol) est dissout dans du DMF anhydre (1 mL) sous argon, à qui de l'HATU (1.2 eq) et de la 2,6-lutidine (1.3 eq) sont ajoutés à température ambiante. Le mélange réactionnel est agité de 15 à 40 min à température ambiante, ensuite une solution d'amine (1.0 eq) dans du DMF (1mL) est ajoutée, et le mélange est agité jusqu'au terme de la réaction (typiquement 4 heures) à température ambiante sous argon. Ensuite le mélange est dilué par l'addition d'une solution d'acide citrique 15% et du EtOAc, puis filtré. Le filtrat est extrait trois fois avec du EtOac, lavé avec des solutions saturées de NaCl et NaHCO₃. La couche organique est séchée (MgSO₄) et évaporée sous pression réduite. Le produit brut est purifié par chromatographie flash pour donner l'amide souhaité.

### 3.4. Procédure générale 3 pour la bromation en utilisant du tribromure de phosphore :

L'alcool est dissout dans du ACN anhydre sous argon, ensuite le PBr3 est ajouté et le mélange réactionnel est chauffé à 60°C sous argon pendant 16 h. Après refroidissement, le mélange réactionnel est dilué avec de l'eau, et extrait trois fois avec de l'EtOAc. Les couches organiques combinées sont lavées avec de la saumure et séchées sur du MgSO₄. L'évaporation du solvant sous pression réduite a donné le composé bromé correspondant désiré.

### 3.5. Acide 3-(bromomethyl)-5-(hydroxymethyl) benzoïque (11)

Le composé **11** est préparé à partir de **10** (317 mg, 0.98 mmol) par la procédure générale **1** pour donner **11** (160 mg, 66%) sous forme d'un solide blanc.

¹H NMR (300 MHz, CDCl₃) : δ(ppm) 8,21 - 7,97 (m, 2H, *H*ₐᵣₒₘ), 7.69 (d, *J* = 2,0 Hz, 1H, *H*ₐᵣₒₘ), 4.64 (d, *J* = 3,0 Hz, 2H, C*H*₂OH), 4,53 (d, *J* = 3,0 Hz, 2H, C*H*₂Br).

¹³C NMR (75 MHz, CDCl₃): δ(ppm) 170,10 (1C, COO), 139,34 (1C, C-5), 137,86 (1C, C-3), 134,91 (1C, *C*H-4), 134,45 (1C, *C*-1), 130,71 (1C, *C*-2), 130,28 (1C, *C*-3), 45,10 (1C, *C*H₂OH), 31,81 (1C, *C*H₂Br).

### 3.6. Méthyle 6-(3-(bromomethyl)-5-(hydroxymethyl)benzamido)hexanoate (12)

Le composé **12** est préparé à partir de **11** (50 mg, 0.20 mmol) par la procédure générale **2** pour donner **12** (41 mg, 54%) sous forme d'un solide blanc.

¹H NMR (300 MHz, CDCl₃) : δ(ppm) 7.78 - 7.50 (m, 3H, *H*ₐᵣₒₘ), 6.35 (s, 1H, NH), 4.60 (d, *J* = 3.1 Hz, 2H, C*H*₂OH), 4.49 (d, *J* = 3.1 Hz, 2H, C*H*₂Br), 3.66 (s, 3H, C*H*₃), 3.47 (dd, *J* = 13.0, 6.9 Hz, 2H, C*H*₂-5), 2.34 (t, *J* = 7.3 Hz, 2H, C*H*₂-1), 1.78 - 1.54 (m, 4H, C*H*₂-2, C*H*₂-3), 1.52 - 1.33 (m, 2H, C*H*₂-4).

¹³C NMR (75 MHz, CDCl₃): δ(ppm) 174.28 (1C, COO), 166.53 (1C, CONH), 139.08 (1C, *C_{Ar}*-5)*,* 138.75 (1C, *C_{Ar}*-3)*,* 136.08 (1C, *C_{Ar}*-1)*,* 131.98 (1C, *C_{Ar}*-2)*,* 127.62 (1C, *C_{Ar}*-2)*,* 127.17 (1C, *C_{Ar}*-6), 51.71 (1C, *C*H₂OH), 45.36 (1C, CH-5), 39.90 (1C, CH-1), 33.88 (1C, *C*H₂Br), 32.19 (1C, CH₃), 29.21 (1C, CH-2), 26.40 (1C, CH-3), 24.37 (1C, CH-4).

### 3.7. Acide 6-(3-(bromomethyl)-5-(hydroxymethyl)benzamido) hexanoïque (13)

Le composé **13** est préparé à partir de **12** (215 mg, 0.58 mmol) par la procédure générale **1** pour donner **13** (160 mg, 77%) sous forme d'un solide blanc électrostatique.

HRMS : m/z calculé : 356,0576 [M + H]+, trouvé : 356,0578.

¹H NMR (300 MHz, DMSO-*d₆*): δ(ppm) 12,0 (bs, 1H, COOH), 8,57 (t, *J* = 5,5 Hz, 1H, NH), 7,92 - 7,61 (m, 3H, *H*ₐᵣₒₘ), 4,90 - 4,67 (m, 4H, C*H*₂Br, C*H*₂OH), 3,24 (dd, *J* = 12,8, 6,8 Hz, 2H, C*H*₂-5), 2,21 (t, *J* = 7,3 Hz, 2H, C*H*₂-1), 1,62 - 1,42 (m, 4H, C*H*₂*-*2, C*H*₂-3), 1,40 - 1.23 (m, 2H, C*H*₂-4).

¹³C NMR (75 MHz, CDCl₃): δ(ppm) 174,50 (1C, COO), 165,21 (1C, CONH), 138,35 (1C, *C_{Ar}*-5)*,* 135,60 (1C, *C_{Ar}*-3), 133,92 (1C, *C_{Ar}-*1), 131,59 (1C, *C_{Ar}*-2), 127,58 (1C, *C_{Ar}*-6), 45,48 (1C, *C*H₂OH), 39,15 (1C, CH-5), 33,62 (2C, CH-1, C*H*₂Br), 28,81 (1C, CH-2), 26,05 (1C, CH-3), 24,27 (1C, CH-4).

### 3.8. Acide 6-(3,5-bis(bromomethyl)benzamido) hexanoïque (14)

Le composé **14** est préparé à partir de **13** (20 mg, 0.006 mmol) par la procédure générale **3** pour donner **14** (24 mg, quantitatif) sous forme d'un solide blanc cassé.

HRMS : m/z calculé : 419,9732 [M + H]+, trouvé : 419,9808.

¹H NMR (300 MHz, DMSO-*d₆*): δ(ppm) 11.8 (bs, 1H, COOH), 8.57 (s, 1H, NH), 7.92 - 7.62 (m, 3H, *H*ₐᵣₒₘ), 4.78 (dd, *J* = 20.8, 3.3 Hz, 4H, 2 x C*H*₂Br), 3.32 - 3.06 (m, 2H+water, C*H*₂-5), 2.21 (t, *J* = 7.3 Hz, 2H, C*H*₂-1), 1.65 - 1.41 (m, 4H, C*H*₂-2, C*H*₂-3), 1.40 - 1.23 (m, 2H, C*H*₂-4).

¹³C NMR (75 MHz, DMSO-*d₆*): δ(ppm) 174.50 (1C, COO), 165.21 (1C, CONH), 138.37 (1C, *C_{Ar}*-5)*,* 135.58 (1C, *C_{Ar}*-3)*,* 132.00 (1C, *C_{Ar}*-1)*,* 131.58 (1C, *C_{Ar}*-2), 127.58 (1C, *C_{Ar}*-6), 45.57 (1C, *C*H₂OH), 39.08 (1C, CH-5), 33.73 (3C, CH-1, C*H*₂Br), 28.89 (1C, CH-2), 26.04 (1C, CH-3), 24.32 (1C, CH-4).

### 3.9. 6-(3,5-bis(bromomethyl)benzamido)hexanamide-Val-Cit-PAB-OH (15)

Le composé **15** est préparé à partir de **14** (124 mg, 0.29 mmol) par la procédure générale **2** pour donner **15** (30 mg, 13%) sous forme d'un solide beige.

HRMS : m/z calculé : 781,1846 [M + H]+, trouvé : 781,1907.

¹H NMR (300 MHz, DMSO-*d₆*): δ(ppm) 9,95 (s, 1H, NH PAB), 8,71 - 8,50 (m, 1H, CONH Cit), 8,10 (d, *J*= 7,5 Hz, 1H, CON*H* Val), 8,04 - 7,55 (m, 3H, *H*ₐᵣₒₘ), 7,55, 7,22 (2d, *J*= 8,5 Hz, 4H, Ar PAB), 6,06 (t, *J* = 5,6 Hz, 1H, N*H* Cit), 5,45 (br s, 2H, N*H*₂ Cit), 5,12 (br s, 1H, OH PAB), 4,82 (s, 4H, 2 x C*H*₂Br), 4,46 - 4,32 (m, 3H, C*H*₂ PAB, *H*_{α} Cit), 4,26 - 4,13 (m, 1H, *H*_{α} Val), 3,24 (dd, *J* = 12,8, 6,6 Hz, C*H*₂-5), 3,10 - 2,84 (m, 2H, NC*H*₂ Cit), 2,29 - 2,07 (m, 2H, C*H*₂-1), 1,97 (dq, *J* = 13,7, 6,6 Hz, 1H, *H*_{β} Val), 1,80-1,05 (m, 10H, C*H*₂C*H*₂ Cit, NCH₂C*H*₂C*H*₂C*H*₂), 0,83 (dd, *J* = 8,9, 7,0 Hz, 6H, C*H*₃ Val).

¹³C NMR (75 MHz, DMSO-*d₆*): δ(ppm) 172,44, 171,29, 170,42, 165,18, 165,02, 158,94, 151,89, 151,12, 139,24, 138,32, 138,28, 137,56, 137,41, 135,59, 135,45, 134,46, 133,79, 132,58, 131,55, 129,41, 128,86, 128,73, 128,60, 127,59, 126,92, 121,31, 120,73, 118,86, 81,78, 62,60, 57,67, 53,08, 48,61, 45,49, 45,36, 38,56, 35,17, 30,37, 29,36, 28,86, 26,79, 26,19, 25,23, 19,26, 18,23.

### Exemple 4: Dérivé de pyridine comme tête de bioconjugaison

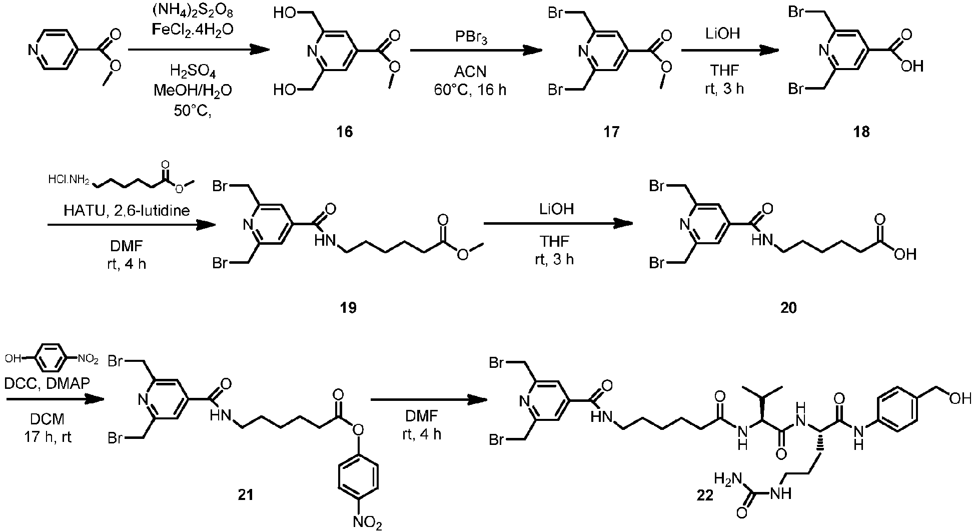

### 4.1. Méthyle 2,6-bis(hydroxymethyl)isonicotinate (16)

Le méthylisonicotinate (2,12 mL, 18 mmol) est dissout sous argon dans un mélange de MeOH/H2O : 1/1 (40 mL), ensuite le H₂SO₄ (78 µl) est ajouté est le mélange est agité pendant 30 min. Ensuite, le persulfate d'ammonium (32 g, 144 mmol) et le chlorure de fer tétrahydraté (0,89 g, 4,5 mmol) sont ajoutés avec précaution à 0°C et le mélange est chauffé à 50 °C toute la nuit. Après refroidissement du mélange réactionnel, la suspension est filtrée et le solide est lavé avec une petite quantité d'EtOAc. Le filtrat est concentré sous pression réduite jusqu'à ce que l'élimination du MeOH soit complète. Le milieu est rendu basique avec du Na₂CO₃ et est ensuite extrait avec du EtOAc. La couche organique est séchée sur du MgSO₄, filtrée et concentrée sous pression réduite. Le produit brut est purifié par chromatographie flash dans un gradient DCM de 10% de MeOH dans le DCM pour donner **16** sous forme d'un solide marron foncé (0,54g, 15%).

¹H NMR (300 MHz, CDCl₃) : δ(ppm) 7,82 (s, 2H, *H*ₐᵣₒₘ), 4,88 (s, 4H, C*H*₂OH), 3,98 (s, 3H, C*H*₃).

¹³C NMR (75 MHz, CDCl₃): δ(ppm) 165,57 (1C, *C*OOCH₃), 160,00 (1C, *C*-1), 150,47 (2C, *C*-3, *C*-5), 118,77 (2C, *C*-2, *C*-6), 64,54 (2C, *C*H₂OH), 52,98 (1C, *C*H₃).

### 4.2. Méthyle 2,6-bis(bromomethyl)isonicotinate (17)

Le composé **17** est préparé à partir de **16** (120 mg, 061 mmol) par la procédure générale **3** pour donner **17** (141 mg, 72%) sous forme d'un solide rose clair.

¹H NMR (300 MHz, CDCl₃): δ(ppm) 7,92 (s, 2H, *H*ₐᵣₒₘ), 4,58 (s, 4H, C*H*₂Br), 3,98 (s, 3H, C*H*₃).

¹³C NMR (75 MHz, CDCl₃): δ(ppm) 164,8 (1C, *C*OOCH₃), 158,0 (1C, *C*-1), 139,8 (2C, *C*-3, *C*-5), 122,3 (2C, *C*-2, *C*-6), 53,0 (1C, *C*H₃), 32,8 (2C, *C*H₂Br).

### 4.3. Acide isonicotinique 2,6-bis(bromomethyl) (18)

Le composé **18** est préparé à partir de **17** (137 mg, 0.42 mmol) par la procédure générale **1** pour donner **18** (133 mg, quantitatif) sous forme d'un solide orange.

HRMS : m/z calculé : 307,88441 [M + H]+, trouvé : 307,8917.

¹H NMR (300 MHz, DMSO-*d₆*): δ(ppm) 7,92 (s, 2H, *H*ₐᵣₒₘ), 4,79 (s, 4H, C*H*₂Br), 3,35 (s, 3H, C*H*₃).

¹³C NMR (75 MHz, DMSO-*d₆*) : δ(ppm) 165,9 (1C, *C*OOCH₃), 158,6 (1C, *C*-1), 141,0 (2C, *C*-3, *C*-5), 122,7 (2C, *C*-2, *C*-6), 34,3 (2C, *C*H₂Br).

### 4.4. Methyl 6-(2,6-bis(bromomethyl)isonicotinamido)hexanoate (19)

Le composé **19** est préparé à partir de **18** (260 mg, 0,84 mmol) par la procédure générale **2** pour donner **19** (218 mg, 59 %) sous forme d'un solide jaune.

HRMS : m/z calculé : 434,9841 [M + H]⁺, trouvé : 434,9906.

¹H NMR (300 MHz, CDCl₃) : δ(ppm) 7,70 (s, 2H, *H*ₐᵣₒₘ), 6,41 (s, 1H, NH), 4,58 (s, 4H, C*H*₂Br), 3,69 (s, 3H, OC*H*₃), 3,50 (dd, *J* = 13,5, 7,0 Hz, 2H, C*H*₂-5), 2,36 (t, *J* = 7,0 Hz, 2H, C*H*₂-1), 1,68 (dq, *J* = 13,5, 7,0 Hz, 4H, C*H*₂-2*,* C*H*₂-4), 1,54 - 1,34 (m, 2H, C*H*₂-3).

¹³C NMR (75 MHz, CDCl₃): δ(ppm) 173,1 (1C, *C*OOCH₃), 157,8 (1C, CONH), 155,0 (2Cp_{yridine}, *C*-4, *C*-6), 120,3 (2Cp_{yridine}, *C*-3, C-5), 51,7 (1C, *C*H₃), 39,8 (1C, *C*H₂-5), 33,6 (1C, *C*H₂-1), 32,9 (2C, *C*H₂Br), 28,8 (1C, *C*H₂-4), 26,1 (1C, *C*H₂-3), 24,0 (1C, *C*H₂-2).

### 4.5. Acide 6-(2,6-bis(bromomethyl)isonicotinamido) hexanoïque (20)

Le composé **20** est préparé à partir de **19** (216 mg, 0,50 mmol) par la procédure générale **1** pour donner **20** (201 mg, 96%) sous forme d'un solide blanc cassé.

HRMS : m/z calculé : 420,9684 [M + H]⁺, trouvé : 420,9748.

¹H NMR (300 MHz, DMSO-*d₆*) : δ(ppm) 12,01 (s, 1H, COOH), 8,83 (t, *J* = 6.2 Hz, 1H, NH), 7,84 (s, 2H, *H*ₐᵣₒₘ), 4,74 (s, 4H, C*H*₂Br), 3.,6 (dd, *J* = 12,6, 6,2 Hz, 2H, C*H*₂-5), 2,21 (t, *J* = 7,3 Hz, 2H, C*H*₂-1), 1,67-1,43 (m, 4H, C*H*₂*-*2*,* C*H*₂-4), 1,54 - 1,34 (dd, *J* = 8,2, 4,6 Hz, 2H, C*H*₂-3).

¹³C NMR (75 MHz, DMSO-*d₆*) : δ(ppm) 174,9 (1C, COOH), 164,3 (1C, CONH), 158,0 (2Cp_{yridine}, *C*-CH₂Br), 144,6 (1C_{pyridine}, *C*-CONH), 121,3 (2Cp_{yridine}, CH), 39,5 (1C, *C*H₂-5), 34,6 (2C, *C*H₂Br), 34,0 (1C, *C*H₂-1), 29,0 (1C, *C*H₂-4), 26,4 (1C, *C*H₂-3), 24,7 (1C, *C*H₂-2).

### 4.6. 4-nitrophenyl 6-(2,6-bis(bromomethyl)isonicotinamido)hexanoate (21)

Sous argon à température ambiante, l'acide **20** (70 mg, 0.17 mmol) est dissout dans du CH₂Cl₂ anhydre (2.1 mL) et à ce mélange on ajoute successivement du DMAP dans des quantités catalytiques, du *p*-nitrophénol (30 mg, 1.3 eq) et du DCC (45 mg, 1.3 eq). Le mélange réactionnel est agité pendant 16 heures à température ambiante sous une atmosphère d'argon, puis ensuite filtré. Le filtrat est concentré sous pression réduite et le produit brut est purifié par chromatographie flash dans un gradient de cyclohexane à 40% de EtOAc dans le cyclohexane pour donner **21** sous forme d'un solide jaune pâle (56 mg, 62%). Le produit est vérifié par ¹HNMR et est utilisé directement pour l'étape suivante.

HRMS : m/z calculé : 541,9848 [M + M]⁺, trouvé : 541,9918.

¹H NMR (300 MHz, CDCl₃) : δ(ppm) 8,34 - 8,21 (m, 2H, H_{arom PNP}), 7,67 (s, 2H, *H*_{arom pyridine}), 7,35 - 7,18 (m, 2H, *H*_{arom PNP}), 6,29 (s, 1H, NH), 4,56 (s, 4H, C*H*₂Br), 3,52 (dd, *J* = 13,0, 7,0 Hz, 1H, C*H*₂-5), 2,66 (t, *J* = 7,0 Hz, 1H, C*H*₂-1), 1,91 - 1,45 (m, 6H, C*H*₂*-*2*,* C*H*₂-3, C*H*₂-4).

### 4.7. 6-(2,6-bis(bromomethyl)isonicotinamido)hexanamide-Val-Cit-PAB-OH (22)

4-nitrophényl 6-(2,6-bis(bromomethyl)isonicotinamdo)hexanoate **21** (56 mg, 0,10 mmol) est dissout dans 2,0 mL de DMF sous une atmosphère d'argon à température ambiante. Après 5 min, le H-Val-Cit-PAB-OH (20 mg, 0,05 mmol) est ajouté, et le mélange réactionnel est agité pendant 5 h. Le DMF est ensuite éliminé par lyophilisation, et le produit brut est purifié par chromatographie flash dans un gradient DCM à 10% de MeOH dans le DCM pour donner **22** sous forme d'un solide jaune pâle (8.5 mg, 20%). Le produit est vérifié par ¹HNMR et est utilisé directement pour l'étape suivante.

HRMS : m/z calculé : 781.1798 [M + M]⁺, trouvé : 782.1873.

¹H NMR (300 MHz, DMSO-*d₆*): δ(ppm) 9,90 (s, 1H, NH PAB), 8,83 (d, *J*= 5,7 Hz, 1H, CON*H* Cit), 8,07 (d, *J*= 7,5 Hz, 1H, CON*H* Val), 7,84 (s, 4H, 2C*H*_{pyridine}), 7,54, 7,22 (2d, *J*= 8,5 Hz, 4H, Ar PAB), 5,98 (br t, *J*= 5,7 Hz, 1H, N*H* Cit), 5,42 (br s, 2H, N*H*₂ Cit), 5,11 (br s, 1H, OH PAB), 4,73 (s, 4H, 2 x C*H*₂Br), 4,48-4,31 (m, 3H, C*H*₂ PAB, *H*_{α} Cit), 4,19 (dd, *J*= 7,0 Hz, *J*= 8,5 Hz, 1H, *H*_{α} Val), 3,48-3,12 (water, C*H*₂-5), 3,06-2,89 (m, 2H, NC*H*₂ Cit), 2,18 (dd, *J* = 14,5, 6,5 Hz, 2H, C*H*₂-1), 2,04-1,86 (m, 1H, *H*_{β} Val), 1,79-1,21 (m, 10H, C*H*₂C*H*₂ Cit, NCH₂C*H*₂C*H*₂C*H*₂), 0,83 (dd, *J* = 8,9, 6,8 Hz, 6H, C*H*₃ Val).

### 4.8. Méthyl chlorhydrate 6 aminohexanoate (23)

Sous une atmosphère d'argon à 0°C, SOCl₂ (1,22 mL, 16,7 mmol) est lentement ajouté à du MeOH (10 ml) et le mélange réactionnel est agité pendant 20 min. A cette solution à 0°C est ajouté l'acide 6-aminocaproique (1,00 g, 7,6 mmol), et le mélange est agité pendant 3,5 h. Les volatils sont éliminés sous pression réduite, et le résidu est recristallisé dans un mélange heptane / EtOAc / MeOH pour atteindre l'ester désiré **23** (1,36 g) dans un rendement quantitatif.

¹H NMR (300 MHz, CDCl₃) : δ(ppm) 7,84 (s, 1H, NH), 3,58 (s, 3H, OC*H*₃), 2,85 - 2,65 (m, 2H, C*H*₂-1), 2,30 (t, *J* = 7,3 Hz, 2H, C*H*₂-1), 1,65 - 1,36 (m, 4H, C*H₂*-2, C*H*₂-4), 1,31 (ddd, *J* = 11,9, 7,3, 2,3 Hz, 2H, C*H*₂-3).

### Exemple 5 : Bioconjugaison - Reconstruction des ponts disulfures

### 5.1. Procédure générale 4 pour la bioconjugaison.

Une solution de l'anticorps désiré est prise. Les ponts disulfures interchaînes sont réduits par le TCEP (vérifié par RP-HPLC), ensuite une solution du linker approprié est ajoutée. Après un temps de réaction approprié (la disparition des chaines légères et lourdes et la reconstruction des ponts disulfures sont suivies par RP-HPLC), l'anticorps brut modifié est purifié sur colonnes de Sephadex PD-10.

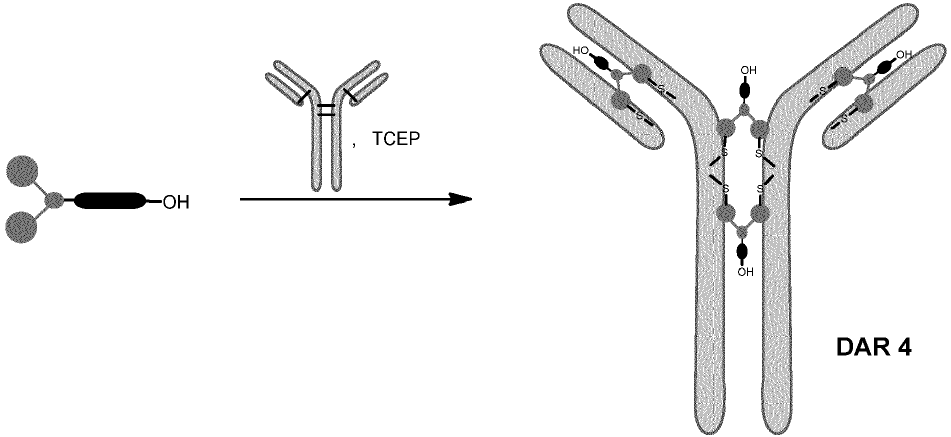

### 5.2. TRASTUZUMAB-(maldiBr-linker7)₄ 24 (non couvert par la présente invention)

Le trastuzumab est réduit puis modifié selon la procédure générale 4, en utilisant le linker 7 non clivable 3,4-dibromomaleimide à 37°C pendant 1h :

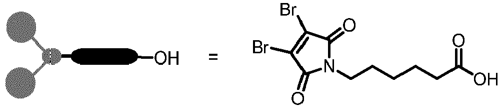

Une solution de TCEP (20éq) dans un tampon PBS est ajoutée à une solution de trastuzumab. Une fraction est prélévée pour analyse afin de confirmer la rupture des ponts disulfures. Le mélange est agité pendant 15 minutes, puis une solution de 6-(3,4-dibromomaleimido)hexanoic Acid (20 éq) dans le DMSO est ajouté. Le mélange réactionnel est laissé sous agitation 30 minutes, puis analysé par RP-HPLC (figure 5).
Une analyse de masse MALDI-TOF (figure 2) permet de confirmer que le mélange réactionnel (vert) contient à 89 % l'espèce résultant du greffage de 4 linkers 6-(3,4-dibromomaleimido)hexanoic Acid sur l'anticorps, la moyenne de greffage est 4.0.

### 5.3. TRASTUZUMAB-(maldiBr-linker8)₄ 25 (non couvert par la présente invention)

Le Trastuzumab est réduit puis modifié selon la procédure générale **4,** en utilisant le linker **8** clivable 3,4-dibromomaleimide à 35°C pendant 1h :

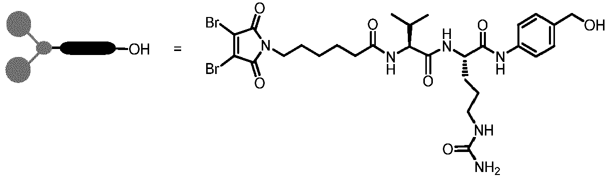

Une solution de TCEP (20éq) dans un tampon PBS est ajoutée à une solution de trastuzumab. Le mélange est agité pendant 15 minutes, puis une solution de 6-(3,4-dibromomaleimido)hexanamide-Val-Cit-PAB-OH (20 éq) dans le DMSO est ajoutée. Le mélange réactionnel est laissé sous agitation 30 minutes, puis après purification sur colonne PD-10 pour supprimer les réactifs chimiques, le mélange est analysé par RP-HPLC (figure 6), et par HIC-HPLC (figure 3).

### 5.4. TRASTUZUMAB-(maldiSPh-linker26)₄ 27 (non couvert par la présente invention)

Le linker **26** est synthétisé d'une manière similaire à celle de WO2013132268, à des fins de comparaison entre la reconstruction des ponts disulfures en utilisant le linker **8** dibromomaleimide ou le linker **26** dithiophenylmaleimide.
Le trastuzumab est réduit puis modifié selon la procédure générale **4,** en utilisant le linker **8** clivable 3,4-dithiophenylmaleimide à 41°C pendant 48h :

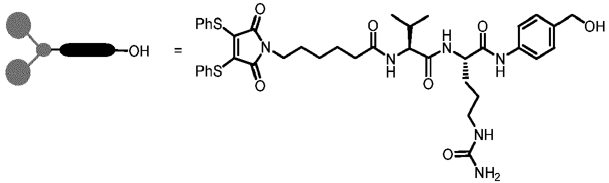

Le mélange réactionnel est ensuite analysé par RP-HPLC (figure 7).

### 5.5. TRASTUZUMAB-(PydiMediBr-linker20)₄ 28

Le trastuzumab est réduit puis modifié selon la procédure générale **4,** en utilisant le linker **20** non clivable acide hexanoique 6-(2,6-bis(bromomethyl)isonicotinamido) à 41°C pendant 24h :

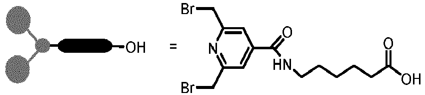

Le mélange réactionnel est ensuite analysé par RP-HPLC (figure 8).

### 5.6. TRASTUZUMAB-(PydiMediBr-linker22)₄ 29

Le trastuzumab est réduit puis modifié selon la procédure générale **4,** en utilisant le linker **22** clivable 6-(2,6-bis(bromomethyl)isonicotinamido)hexanamide-Val-Cit-PAB-OH à 41 °C pendant 24 h :

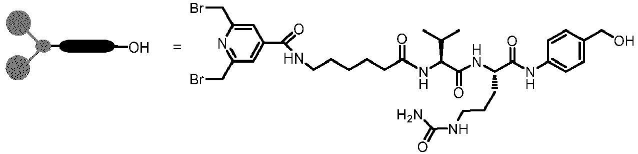

Le mélange réactionnel est ensuite analysé par RP-HPLC (figure 9).

### 5.7. TRASTUZUMAB-(PhdiMediBr-linkerl5)₄ 30

Le trastuzumab est réduit puis modifié selon la procédure générale **4,** en utilisant le linker clivable **15** 6-(3,5-bis(bromomethyl)benzamido)hexanamide-Val-Cit-PAB-OH à 41°C pendant 72h :

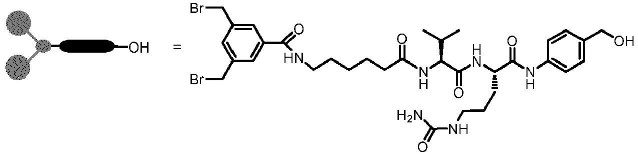

Le mélange réactionnel est ensuite analysé par RP-HPLC (figure 10).

### 5.8. RITUXIMAB-(maldiBr-linker7)₄ 31

Le rituximab est réduit puis modifié selon la procédure générale **4,** en utilisant le linker non clivable **7** à 37°C pendant 1h :

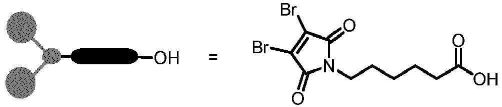

Le mélange réactionnel est ensuite analysé par RP-HPLC (figure 11).

### 5.9. TRASTUZUMAB-(maldiBr-linker9-MMAE)₄ 32 (non couvert par la présente invention)

Le trastuzumab est réduit puis modifié selon la procédure générale **4,** en utilisant le linker **9-MMAE** clivable 6-(3,4-dibromomaleimido)hexanamide-Val-Cit-PAB-MMAE à 37°C pendant 24h : 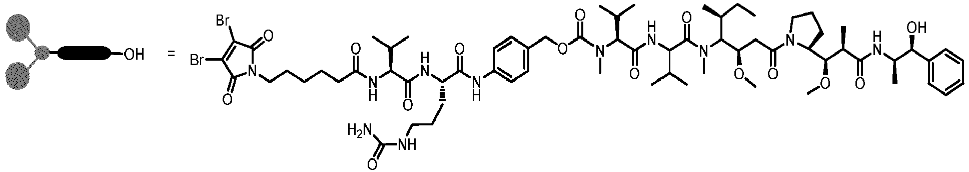 Le mélange réactionnel est ensuite analysé par RP-HPLC (figure 12).

### 5.10. Données expérimentales supplémentaires pour données analytiques

### a- RP-HPLC

Les ADC ont été analysés dans des conditions dénaturantes et réductrices, avec une colonne AERIS WIDEPORE 3,6 µM XB-C8 (250 x 4,6 mm). Le débit était de 1 mL/min, la température du four de 80°C, avec une quantité d'injection de 20 µL par échantillon. Concernant la méthode d'élution : avec deux solvants : A était 0.05% acide trifluoroacétique dans l'eau et B : 0.04% acide trifluoroacétique dans l'acétonitrile. La méthode était la suivante: 25% B isocratique pendant 3 min, un gradient linéaire de 25 min de 25 à 50% B, un gradient linéaire de 2 min de 50 à 95% B, un gradient linéaire de 1 min de 95 à 25% B, puis 25% B isocratique pendant 8 min.

### b- HIC-HPLC

Les ADC ont été analysés dans des conditions non-dénaturantes, avec une colonne TOSOH BIOSCIENCE 2,5 µM T SKgel butyl-NPR (100 x 4,6 mm). Le débit était de 1 mL/min, la température du four de 80°C, avec une quantité d'injection de 30 µL par échantillon. Concernant la méthode d'élution avec deux solvants : A : 1,5 M (NH₄)₂SO₄ dans un tampon phosphate PBS 50 mM (pH 7) et B : mélange tampon phosphate PBS 50 mM / *i*PrOH : 80/20. La méthode était la suivante : un gradient linéaire de 44 min de 0 % B à 80 % B, puis un gradient linéaire de 1 min de 80 à 0% B, puis 0 % B isocratique pendant 10 min.

## Revendications

1. Produit **caractérisé en ce qu'**il répond à la formule I choisie parmi les formules IB2 et IA2: 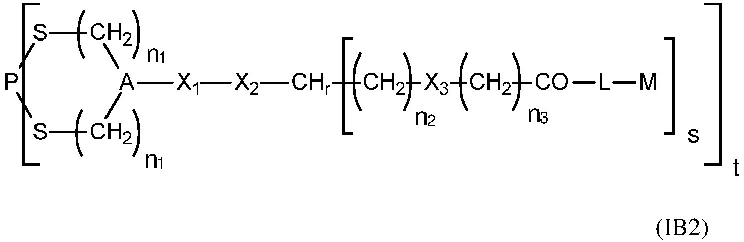 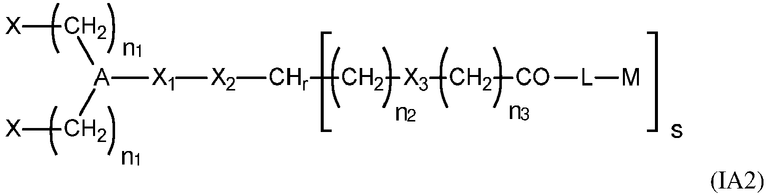 formules IA2 et IB2 dans lesquelles :
le groupe : 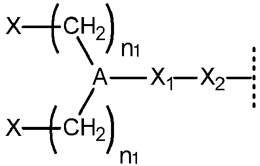 est choisi parmi les radicaux : 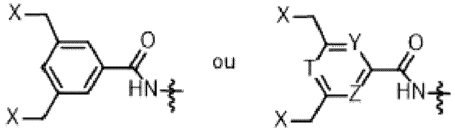 et **en ce que** le groupe : 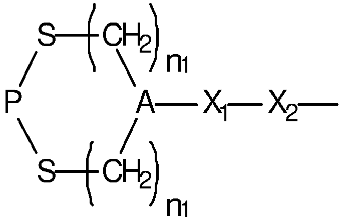 est choisi parmi les radicaux : 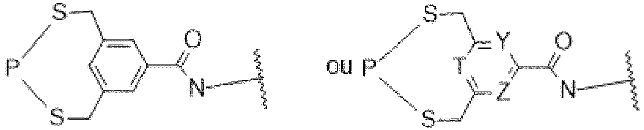
X est Br ou I,
dans la triade d'atomes (T,Y,Z) chacun de ces atomes représente indépendamment des deux autres atomes, un atome de carbone ou d'azote, en particulier T est un atome d'azote et Y et Z représentent CH,
X₃ est un oxygène ou une simple liaison
s est égal à 1, 2 ou 3
r est égal à 0, 1 ou 2
étant entendu que la somme r+s est égale à 3
n₂ est un entier égal à 1, 2 ou 3 et n₃ est un entier égal à 1, 2 ou 3 - L-M est un radical dans lequel, L représente un corps de linker et M est un médicament cytotoxique choisi parmi un agent chimiothérapeutique ou une toxine,
P est une protéine comprenant au moins un pont disulfure et
t représente un entier de 1 à 15,
ainsi que les dérivés de la ou des fonctions acides carboxyliques choisis parmi les sels, les esters ou les amides.

2. Produit selon la revendication 1, dans lequel le médicament M est sélectionné dans le groupe constitué de la duocarmycine et ses analogues, les dolastatines, la combretastatine, la calichéamicine, la N-acétyl-γ-calichéamycine (CMC), un dérivé de calichéamycine, la maytansine et ses analogues, DM-I, l'auristatine E, l'auristatine EB (AEB), l'auristatine EFP (AEFP), la monométhyle d'auristatine E (MMAE), la monométhyle d'auristatine F (MMAF), la tubulysine, le disorazole, les epothilones, le Paclitaxel, le docetaxel, le Topotecan, l'echinomycine, l'estramustine, la cemadotine, l'eleutherobine, la methopterine, l'actinomycine, la daunorubicine, les conjugués de daunorubicine, la mitomycine C, la mitomycin A, la vincristine, l'acide rétinoïque, la camptothécine, un dérivé de la camptothécine, le SN38, la maytansine, un dérivé de type maytansinoïde, le DM1, le DM4, le TK1, l'amanitin, une pyrrolobenzodiazépine, un dimère de pyrrolobenzodiazépine, le méthotrexate, l'ilomédine, l'aspirine, un IMIDs, le lénalidomide, le pomalidomide ou le médicament M est une toxine choisie parmi l'exotoxine de pseudomonas (PE), la deBouganin, la Bouganin, la toxine diphtérique (DT) et la ricine.

3. Produit selon la revendication 1 répondant à la formule (IB2). 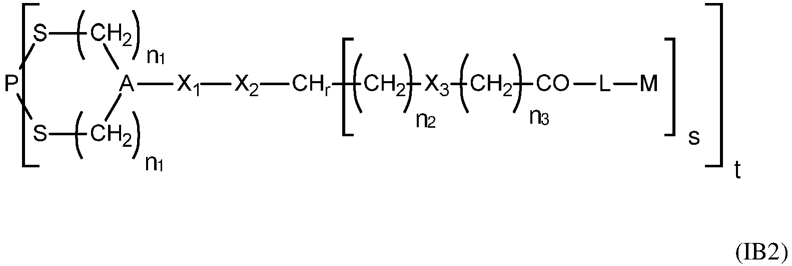 dans laquelle X, A, X1, X2, X3, n₁, n₂,n₃, r, s, t, L, P et M ont la signification indiquée à la revendication 1.

4. Produit selon l'une des revendications précédentes dans lequel la protéine P comprenant au moins un pont disulfure, est un anticorps ou un fragment d'anticorps, une protéine de fusion comportant un anticorps ou un fragment d'anticorps, une albumine, ou un peptide.

5. Produit selon la revendication 4 dans lequel l'anticorps est un anticorps monoclonal chimérique, humanisé ou humain, monospécifique ou bispécifique, une protéine de fusion comportant un anticorps ou un fragment d'anticorps choisi parmi le groupe constitué de: Fab, F(ab)'2, Fc, F'c, pFc', ScFv, Fv, Fd, Fabc, diabody, minibody, ScFv-Fc ou ScFv-Fv.

6. Produit selon la revendication 4 ou 5, dans lequel l'anticorps ou le fragment d'anticorps est dirigé contre un antigène de tumeur ou d'inflammation, l'anticorps ou le fragment d'anticorps étant dirigé
soit contre l'un des antigènes du cluster de différentiation (CD), dont le numéro d'identification varie entre le CD1a et le CD363 et de préférence est choisi parmi CD3, CD4, CD13, CD19, CD20, CD21, CD22, CD25, CD30, CD31, CD33, CD34, CD37, CD39, CD40, CD44, CD47, CD52, CD56, CD66e, CD70, CD72, CD73, CD74, CD79, CD79b, CD80, CD86, CD117, CD138, CD194, CD205, CD227 ou CD248
soit l'anticorps ou le fragment d'anticorps est dirigé contre l'un des antigènes choisis dans la liste formée par CA125, G250, GD2, HLA-DRβ, MUC1, VEGF, VEGFA, VEGF-R1/2, TRAIL-R2 (DR5), EpCAM, GPIIb, GPIIIa, TNF alpha, TNFR, TNT, Lewis Y, EGFR, HER-2, HER-3, HER-3 MM-111, HER-4, homodimère ou hétérodimère entre des membres de la famille erbbₙ, ₙ étant un entier compris entre 1 et 4, AXL, Protéine F, IgE-Fc, VEGF-A, intégrine 4, intégrine alpha4beta7, intégrine alphaV, C5, IL-6R, IL-6Ralpha, IL12, IL15, IL18, IL23, IL-lbeta, IL-1, TPO-R, GPNMB, PSMA, PSA, PAP, PSM, intégrine alphaᵥ, Cripto, TACSTD2 (TROP2 ou EGP1), CEA, Folate receptor 1, Mucin 16, Endothelin Receptor ETB, STEAP1, SLC44A4 (AGS-5), Nectin 4, AGS-16, Guanylyl cyclase C, Mucin 1, EGFRvIII, Mesothelin, IL2R, A33, Can, VEGF-B, VEGF-C, VEGF-D, VEGF-E, VEGFR-1, VEGFR-2, VEGFR-3, TGFbeta, TGFbetaR, FGF, FGF8b, FGFR, PDGF, PDGFR, PDGFRalpha, PDGFRbeta, Ang-1, Ang-2, intégrine, α2β1, alpha-v-beta3, alpha-v-beta5, alpha3beta1, alpha6beta4, alpha2beta1, anti-intégrine alpha4, RANK-L, BLyS, c-MET, DR, DR10, TCRalpha,beta, ICOS, CTLA-4, CAIX (MN), EphA2, CA6, CA6 de l'ovaire, CA6 cervical, CA6 du sein, angiopoiétine-2, Cripto, ENPP3, Mesothelin, FOLR1, Nectin-4, TIM-1, Muc-16, Tissue Factor, LIV-1, GM2, alpha5 integrin, TLR-7, PD-1, AFP, CA125 (MUC16), Sialyl Lewis^{Y}, CAMPATH-1, HLA-DR, anti-idiotype, carcinoembryonic antigen (CEA), TAG-72, Folate-binding protein, A33, G250, gangliosides (incluant GD2, GD3, GM2), Le^{Y}, collagen 4 (collagen IV), collagen 18 (collagen XVIII), SC6, CA-125, CA19-9, p185^{HER2}, de2-7 EGFR, Fibroblast activation protein (FAP), Tenascin, metalloproteinases, Endosialin, Carbonic anhydrase, Galectin 9, Aldolase A, eIFgamma4, Tyrosinase, Galectin 4, HERKV-K10, p53, NY-LU-12, Restin, NY-CO-38, MAGE-1, MAGE-4a, SSX2, NY-ESO-1, SCP-1, HGFR, PTK 7, CCK-4, PDGFR, PTP-LAR, CDCP1, CADM1, IGSF4, Lu, BCAM, CEACAM6, JAM-A, PTGFRN (CD9P-1), MCAM, MUC18, MCP, EMMPRIN, TfR, TRAILR2, C1qR, hTERT, Survivin, MDM2, CYP1B1, Melan-A, MART-1, MART-2, Melanosomal proteins, gp100, neo-PAP, CDC27, MAGEs, WT1, MUM-1, MUM-2, MUM-3, BRAF, TPI, fibronectin, K-ras, beta-catenin, CDK4, caspase-8, p14^{ARF}, p16^{INK4a}, TGFβRII, bcr-abl, SYT-SSX, TRP-1, TRP-2, GnT-V, tyrosinase, FGF5, TEL-AML1, Proteinase 3, HER2/neu, AFP, MUC-1, EBV-EBNA, HTLV-1 tax, HPV16-E7, mutated HLA-A2, HA1, SART3, GnT-V, CEACAM5, AGS-16, GPNMB, ESAT-6, RANK, CanAg, fibrin, TF, PRAME, CA19-9, CA50, CA125, CA195, CAM17.1/WGA, AFP, beta-MG, DU-PAN2, HE4, b-2 microglobulin, transferrin, transthyretin, ApoA1, TROP-2, CTLA-4, GITR, PD-1, PD-L1, c-KIT, CD11b-CD18 integrin heterodimer, DNA/Histon H1, Folate, EpCAM, Tenascin-c, ECM (proteoglycan ou fibronectin), fibrinogen, SV40 large T antigen, SC6-Ag, SC-Ag, DR4 (death receptor 4), DR5, ESA, mucin, hPAM4, hRS7, HLA-DR, ou CCR4, l'antigène de formule générale MTX1, MTX2, PECAM, thrombomoduline, Tn, cathepsin D, TYRO-3, MER
soit l'anticorps ou le fragment d'anticorps est dirigé contre un complexe PF4/héparine.

7. Produits de formule IB2 selon la revendication 1 pour leur utilisation dans le traitement d'une tumeur notamment le cancer colorectal, l'hépatocarcinome, un cancer du poumon, le cancer du pancréas, le cancer du poumon non à petites cellules, le cancer du poumon à petites cellules, le cancer colorectal, le cancer colorectal K-RAS muté, un cancer du sein, le cancer du sein triple-négatif, le cancer du sein métastatique, le cancer du foie, le cancer tête et cou, la maladie de Castleman, le cancer de la thyroïde, le médulloblastome, le glioblastome multiforme, le gliome, le sarcome, l'astrocytome anaplastique, le cancer du rein, le cancer de l'estomac, l'ascite maligne, le cancer de la prostate (métastatique ou non), les tumeurs solides, la leucémie aiguë myéloïde, la leucémie lymphocytaire chronique, le mélanome, le myélome, le myélome multiple les lymphomes, le lymphome du manteau, le lymphome hodgkinien, le lymphome non-hodgkinien, le lymphome folliculaire ou pour leur utilisation dans le traitement d'une maladie inflammatoire ou auto-immune notamment le rejet de greffe, la polyarthrite rhumatoïde, la maladie de Crohn, le psoriasis, la sclérose en plaques, les syndromes périodiques associés à la cryopyrine, le purpura thrombopénique.

8. Produit selon l'une des revendications précédentes dans lequel les dérivés de la ou des fonctions acides carboxyliques est l'amide formée avec le butanimide.

9. Produit selon l'une des revendications précédentes dans lequel M est la monométhyle d'auristatine E ou la monométhyle d'auristine F.

## Patentansprüche

1. Produkt, **dadurch gekennzeichnet, dass** es der Formel I entspricht, ausgewählt aus den Formeln IB2 und IA2: 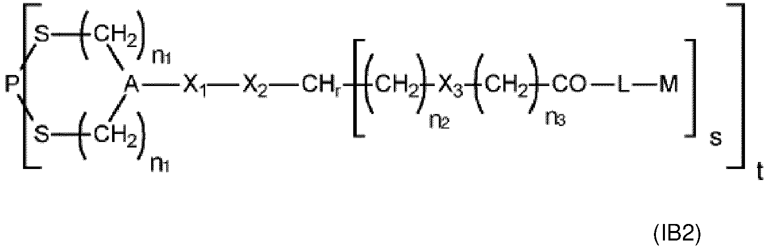 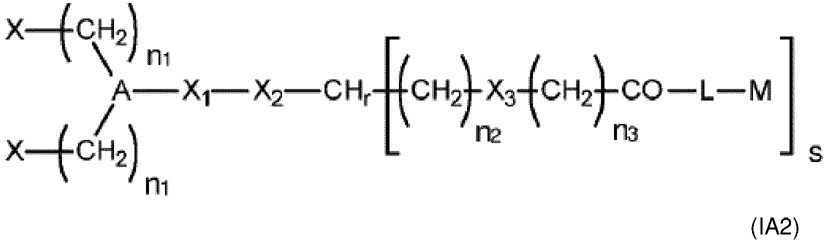 wobei in den Formeln IA2 und IB2 gilt:
die Gruppe: 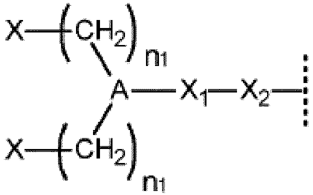 ist ausgewählt aus den Radikalen: 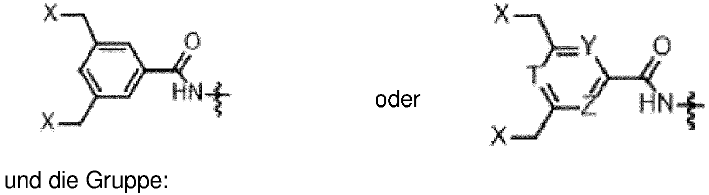 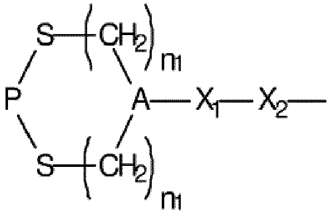 ist ausgewählt aus den Radikalen: 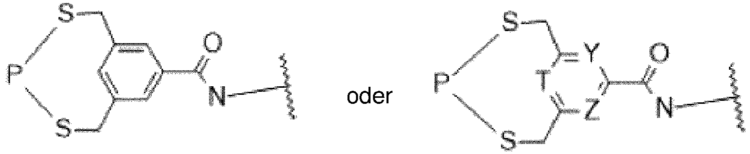 wobei X Br oder I ist,
in der Triade von Atomen (T,Y,Z) jedes dieser Atome unabhängig von den beiden anderen Atomen ein Kohlenstoff- oder Stickstoffatom darstellt, insbesondere T ein Stickstoffatom ist und Y und Z CH darstellen,
X₃ Sauerstoff oder eine Einfachbindung ist
s gleich 1, 2 oder 3 ist und
r gleich 0, 1 oder 2 ist
mit der Maßgabe, dass die Summe r+s gleich 3 ist,
n₂ eine ganze Zahl gleich 1, 2 oder 3 und n₃ eine ganze Zahl gleich 1, 2 oder 3 ist - L-M ein Radikal ist, wobei L einen Linkerkörper darstellt und M ein zytotoxischer Wirkstoff ist, ausgewählt aus einem Chemotherapeutikum oder einem Toxin,
P ein Protein mit mindestens einer Disulfidbrücke ist, und
t eine ganze Zahl von 1 bis 15 darstellt,
sowie die Derivate der Carbonsäurefunktion(en) ausgewählt aus Salzen, Estern oder Amiden sind.

2. Produkt nach Anspruch 1, wobei der Wirkstoff M ausgewählt ist aus der Gruppe, bestehend aus Duocarmycin und seinen Analoga, Dolastatinen, Combretastatin, Calicheamicin, N-Acetyl-γ-Calicheamycin (CMC), einem Derivat von Calicheamycin, Maytansin und seinen Analoga, DM-I, Auristatin E, Auristatin EB (AEB), Auristatin EFP (AEFP), Monomethyl-Auristatin E (MMAE), Monomethyl-Auristatin F (MMAF), Tubulysin, Disorazol, Epothilone, Paclitaxel, Docetaxel, Topotecan, Echinomycin, Estramustin, Cemadotin, Eleutherobin, Methopterin, Actinomycin, Daunorubicin, Daunorubicin-Konjugate, Mitomycin C, Mitomycin A, Vincristin, Retinolsäure, Camptothecin, ein Derivat von Camptothecin, SN38, Maytansin, ein Maytansinoid-Derivat, DM1, DM4, TK1, Amanitin, ein Pyrrolobenzodiazepin, Pyrrolobenzodiazepin-Dimer, Methotrexat, Ilomedin, Aspirin, IMIDs, Lenalidomid, Pomalidomid oder der Wirkstoff M ist ein Toxin, ausgewählt aus Pseudomonas-Exotoxin (PE), DeBouganin, Bouganin, Diphtherietoxin (DT) und Ricin.

3. Produkt nach Anspruch 1 entsprechend der Formel (IB2): 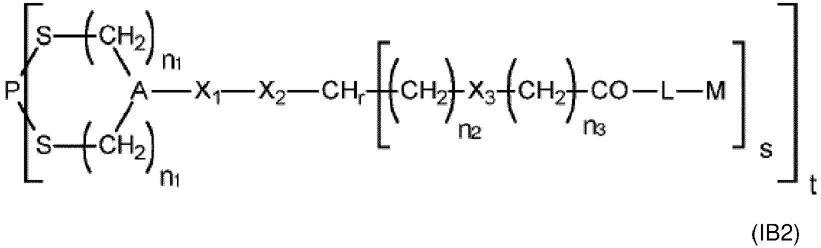 wobei X, A, X1, X2, X3, n₁, n₂, n₃, r, s, t, L, P und M die in Anspruch 1 angegebenen Bedeutungen aufweisen.

4. Produkt nach einem der vorhergehenden Ansprüche, wobei es sich bei dem Protein P, das mindestens eine Disulfidbrücke umfasst, um einen Antikörper oder ein Antikörperfragment, ein Fusionsprotein mit einem Antikörper oder Antikörperfragment, ein Albumin oder ein Peptid handelt.

5. Produkt nach Anspruch 4, wobei es sich bei dem Antikörper um einen chimären, humanisierten oder humanen, monospezifischen oder bispezifischen monoklonalen Antikörper, ein Fusionsprotein mit einem Antikörper oder Antikörperfragment, ausgewählt aus der Gruppe, bestehend aus: Fab, F(ab)'2, Fc, F'c, pFc', ScFv, Fv, Fd, Fabc, Diabody, Minibody, ScFv-Fc oder ScFv-Fv, handelt.

6. Produkt nach Anspruch 4 oder 5, wobei der Antikörper oder das Antikörperfragment gegen ein Tumor- oder Entzündungsantigen gerichtet ist, wobei der Antikörper oder das Antikörperfragment gerichtet ist:
entweder gegen eines der Antigene des Differenzierungsclusters (CD), dessen Identifikationsnummer zwischen CD1a und CD363 variiert und vorzugsweise ausgewählt ist aus CD3, CD4, CD13, CD19, CD20, CD21, CD22, CD25, CD30, CD31, CD33, CD34, CD37, CD39, CD40, CD44, CD47, CD52, CD56, CD66e, CD70, CD72, CD73, CD74, CD79, CD79b, CD80, CD86, CD117, CD138, CD194, CD205, CD227 oder CD248
oder der Antikörper oder das Antikörperfragment ist gerichtet gegen eines der Antigene, ausgewählt aus der Liste, bestehend aus CA125, G250, GD2, HLA-DRβ, MUC1, VEGF, VEGFA, VEGF-R1/2, TRAIL-R2 (DR5), EpCAM, GPIIb, GPIIIa, TNF alpha, TNFR, TNT, Lewis Y, EGFR, HER-2, HER-3, HER-3 MM-111, HER-4, Homodimer oder Heterodimer zwischen Mitgliedern der erbbₙ-Familie, wobei n eine ganze Zahl zwischen 1 und 4 ist, AXL, Protein F, IgE-Fc, VEGF-A, Integrin 4, alpha4beta7-Integrin, alphaV-Integrin, C5, IL-6R, IL-6Ralpha, IL12, IL15, IL18, IL23, IL-1beta, IL-1, TPO-R, GPNMB, PSMA, PSA, PAP, PSM, alphaᵥ Integrin, Cripto, TACSTD2 (TROP2 oder EGP1), CEA, Folatrezeptor 1, Mucin 16, Endothelin-Rezeptor ETB, STEAP1, SLC44A4 (AGS-5), Nektin 4, AGS-16, Guanylylcyclase C, Mucin 1, EGFRvIII, Mesothelin, IL2R, A33, Can, VEGF-B, VEGF-C, VEGF-D, VEGF-E, VEGFR-1, VEGFR-2, VEGFR-3, TGFbeta, TGFbetaR, FGF, FGF8b, FGFR, PDGF, PDGFR, PDGFRalpha, PDGFRbeta, Ang-1, Ang-2, Integrin, α2β1, alpha-v-beta3, alpha-v-beta5, alpha3beta1, alpha6beta4, alpha2beta1, alpha4 Antiintegrin, RANK-L, BLyS, c-MET, DR, DR10, TCRalpha,beta, ICOS, CTLA-4, CAIX (MN), EphA2, CA6, Ovarial-CA6, Zervikal-CA6, Brust-CA6, Angiopoietin-2, Cripto, ENPP3, Mesothelin, FOLR1, Nectin-4, TIM-1, Muc-16, Tissue Factor, LIV-1, GM2, alpha5-Integrin, TLR-7, PD-1, AFP, CA125 (MUC16), Sialyl Lewis^{Y}, CAMPATH-1, HLA-DR, Anti-Idiotyp, carcinoembryonales Antigen (CEA), TAG-72, Folat-bindendes Protein, A33, G250, Ganglioside (einschließlich GD2, GD3, GM2), Le^{Y}, Kollagen 4 (Kollagen IV), Kollagen 18 (Kollagen XVIII), SC6, CA-125, CA19-9, p185^{HER2} , de2-7 EGFR, Fibroblasten-Aktivierungsprotein (FAP), Tenascin, Metalloproteinasen, Endosialin, Carbonic anhydrase, Galectin 9, Aldolase A, elFgamma4, Tyrosinase, Galectin 4, HERKV-K10, p53, NY-LU-12, Restin, NY-CO-38, MAGE-1, MAGE-4a, SSX2, NY-ESO-1, SCP-1, HGFR, PTK 7, CCK-4, PDGFR, PTP-LAR, CDCP1, CADM1, IGSF4, Lu, BCAM, CEACAM6, JAM-A, PTGFRN (CD9P-1), MCAM, MUC18, MCP, EMMPRIN, TfR, TRAILR2, C1qR, hTERT, Survivin, MDM2, CYP1B1, Melan-A, MART-1, MART-2, Melanosomale Proteine, gp100, neo-PAP, CDC27, MAGEs, WT1, MUM-1, MUM-2, MUM-3, BRAF, TPI, Fibronectin, K-ras, beta-Catenin, CDK4, Caspase-8, p14^{ARF}, p16^{INK4a}, TGFβRII, bcr-abl, SYT-SSX, TRP-1, TRP-2, GnT-V, Tyrosinase, FGF5, TEL-AML1, Proteinase 3, HER2/neu, AFP, MUC-1, EBV-EBNA, HTLV-1 tax, HPV16-E7, mutiertes HLA-A2, HA1, SART3, GnT-V, CEACAM5, AGS-16, GPNMB, ESAT-6, RANK, CanAg, Fibrin, TF, PRAME, CA19-9, CA50, CA125, CA195, CAM17. 1/WGA, AFP, beta-MG, DU-PAN2, HE4, b-2-Mikroglobulin, Transferrin, Transthyretin, ApoA1, TROP-2, CTLA-4, GITR, PD-1, PD-L1, c-KIT, CD11b-CD18-Integrin-Heterodimer, DNA/Histon H1, Folat, EpCAM, Tenascin-c, ECM (Proteoglykan oder Fibronektin), Fibrinogen, SV40 large T antigen, SC6-Ag, SC-Ag, DR4 (death receptor 4), DR5, ESA, Mucin, hPAM4, hRS7, HLA-DR oder CCR4, das Antigen der allgemeinen Formel MTX1, MTX2, PECAM, Thrombomodulin, Tn, Cathepsin D, TYRO-3, MER oder der Antikörper oder das Antikörperfragment ist gerichtet gegen einen PF4/Heparin-Komplex.

7. Produkte der Formel IB2 nach Anspruch 1 zur Verwendung bei der Behandlung eines Tumors, insbesondere von kolorektalem Krebs, Hepatokarzinom, Lungenkrebs, Bauchspeicheldrüsenkrebs, nicht-kleinzelligem Lungenkrebs, kleinzelligem Lungenkrebs, kolorektalem Krebs, mutiertem K-RAS Kolorektalkrebs, Brustkrebs, dreifach negativem Brustkrebs, metastasiertem Brustkrebs, Leberkrebs, Kopf- und Halskrebs, Castleman-Krankheit, Schilddrüsenkrebs, Medulloblastom, Glioblastoma multiforme, Gliom, Sarkom, anaplastischem Astrozytom, Nierenkrebs, Magenkrebs, malignem Aszites, Prostatakrebs (metastasiert oder nicht metastasiert), soliden Tumoren, akuter myeloischer Leukämie, chronischer lymphatischer Leukämie, Melanom, Myelom, multiplem Myelom, Lymphomen, Mantellymphom, Hodgkin-Lymphom, Non-Hodgkin-Lymphom, follikulärem Lymphom oder zur Verwendung bei der Behandlung von Entzündungs- oder Autoimmunerkrankungen, insbesondere bei Transplantatabstoßung, rheumatoider Arthritis, Morbus Crohn, Psoriasis, multipler Sklerose, periodischen Kryopyrin-assoziierten Syndromen und thrombozytopenischer Purpura.

8. Produkt nach einem der vorhergehenden Ansprüche, wobei es sich bei den Derivaten der Carbonsäurefunktion(en) um das mit Butanimid gebildete Amid handelt.

9. Produkt nach einem der vorhergehenden Ansprüche, wobei es sich bei M um das Monomethyl von Auristatin E oder das Monomethyl von Auristatin F handelt.

## Claims

1. Product **characterized in that** it complies with formula I chosen from formulae IB2 and IA2: 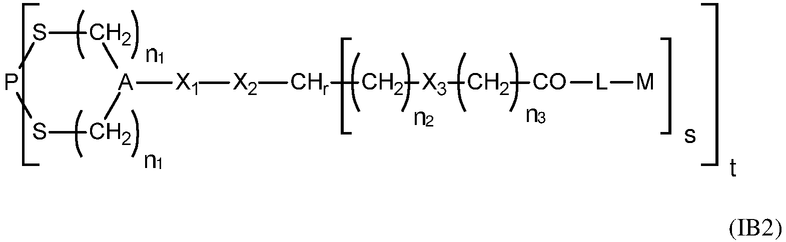 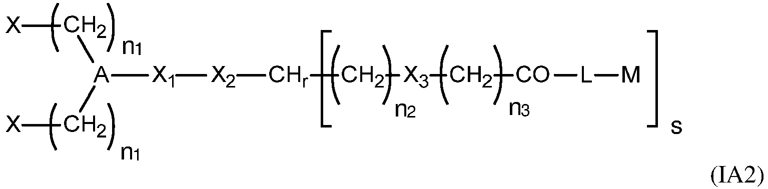 formulae IA2 and IB2 wherein:
the group: 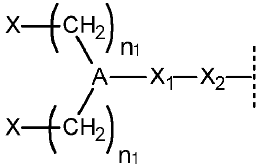 is chosen from the radicals: 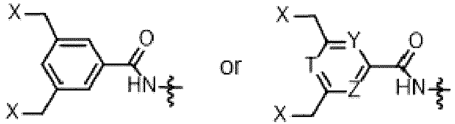 and **in that** the group: 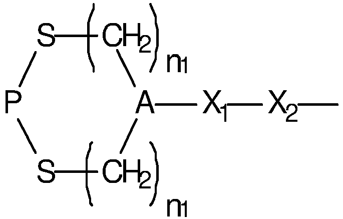 is chosen from the radicals: 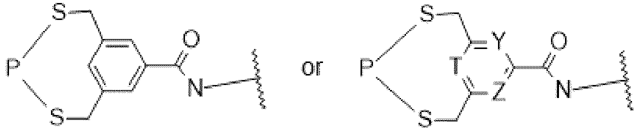
X is Br or I,
in the triad of atoms (T, Y, Z) each of these atoms represents, independently of the two other atoms, a carbon or nitrogen atom, in particular T is a nitrogen atom and Y and Z represent CH,
X₃ is oxygen or a single bond
s is equal to 1, 2 or 3,
r is equal to 0, 1 or 2
provided that the sum r+s is equal to 3
n₂ is an integer equal to 1, 2 or 3 and n₃ is an integer equal to 1, 2 or 3
-L-M is a radical wherein L represents a linker body and M is a cytotoxic drug chosen from a chemotherapeutic agent or a toxin,
P is a protein comprising at least one disulfide bridge and
t represents an integer from 1 to 15,
as well as the derivatives of the carboxylic acid function(s) chosen from salts, esters or amides.

2. Product according to claim 1, wherein drug M is selected from the group consisting of duocarmycin and its analogues, dolastatins, combretastatin, calicheamicin, N-acetyl-γ-calicheamycin (CMC), a calicheamycin derivative, maytansine and its analogues, DM-I, auristatin E, auristatin EB (AEB), auristatin EFP (AEFP), monomethylauristatin E (MMAE), monomethylauristatin F (MMAF), tubulysine, disorazole, epothilones, Paclitaxel, docetaxel, Topotecan, echinomycin, estramustine, cemadotine, eleutherobin, methopterin, actinomycin, daunorubicin, daunorubicin conjugates, mitomycin C, mitomycin A, vincristine, retinoic acid, camptothecin, a camptothecin derivative, SN38, maytansine, a derivative of the maytansinoid-type, DM1, DM4, TK1, amanitin, a pyrrolobenzodiazepine, a pyrrolobenzodiazepine dimer, methotrexate, ilomedine, aspirin, an IMIDs, lenalidomide, pomalidomide or drug M is a toxin chosen from pseudomonas exotoxin (PE), deBouganin, Bouganin, diphtheria toxin (DT) and ricin.

3. Product according to claim 1 complying with formula (IB2) 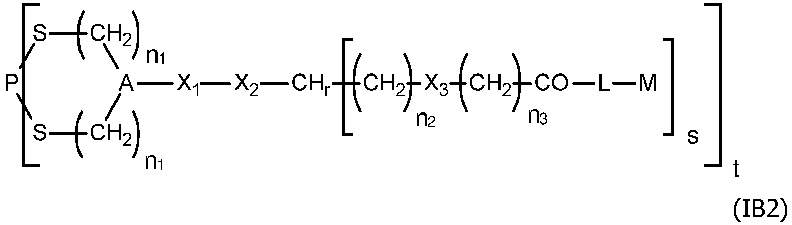 wherein X, A, X1, X2, X3, n₁, n₂, n₃, r, s, t, L, P and M have the meaning indicated in claim 1.

4. Product according to one of the preceding claims wherein protein P comprising at least one disulfide bridge is an antibody or an antibody fragment, a fusion protein comprising an antibody or an antibody fragment, an albumin, or a peptide.

5. Product according to claim 4 wherein the antibody is a chimeric, humanized or human, monospecific or bispecific monoclonal antibody, a fusion protein comprising an antibody or an antibody fragment chosen from the group consisting of: Fab, F(ab)'2, Fc, F'c, pFc', ScFv, Fv, Fd, Fabc, diabody, minibody, SeFv-Fc or ScFv-Fv.

6. Product according to claim 4 or 5, wherein the antibody or the antibody fragment is directed against a tumor or inflammation antigen, the antibody or the antibody fragment being directed either against one of the antigens of the cluster of differentiation (CD), the identification number of which varies between CD1a and CD363 and is preferably chosen from CD3, CD4, CD13, CD19, CD20, CD21, CD22, CD25, CD30, CD31, CD33, CD34, CD37, CD39, CD40, CD44, CD47, CD52, CD56, CD66e, CD70, CD72, CD73, CD74, CD79, CD79b, CD80, CD86, CD117, CD138, CD194, CD205, CD227 or CD248 either the antibody or the antibody fragment is directed against one of the antigens chosen from the list formed by CA125, G250, GD2, HLA-DRβ, MUC1, VEGF, VEGFA, VEGF-R1/2, TRAIL-R2 (DR5), EpCAM, GPIIb, GPIIIa, TNF alpha, TNFR, TNT, Lewis Y, EGFR, HER-2, HER-3, HER-3 MM-111, HER-4, homodimer or heterodimer between members of the family erbbₙ, ₙ being an integer comprised between 1 and 4, AXL, Protein F, IgE-Fc, VEGF-A, integrin 4, integrin alpha4beta7, integrin alphaV, C5, IL-6R, IL-6Ralpha, IL12, IL15, IL18, IL23, IL-1beta, IL-1, TPO-R, GPNMB, PSMA, PSA, PAP, PSM, integrin alphaᵥ, Cripto, TACSTD2 (TROP2 or EGP1), CEA, Folate receptor 1, Mucin 16, Endothelin Receptor ETB, STEAP1, SLC44A4 (AGS-5), Nectin 4, AGS-16, Guanylyl cyclase C, Mucin 1, EGFRvIII, Mesothelin, IL2R, A33, Can, VEGF-B, VEGF-C, VEGF-D, VEGF-E, VEGFR-1, VEGFR-2, VEGFR-3, TGFbeta, TGFbetaR, FGF, FGF8b, FGFR, PDGF, PDGFR, PDGFRalpha, PDGFRbeta, Ang-1, Ang-2, integrin, α2β1, alpha-v-beta3, alpha-v-beta5, alpha3beta1, alpha6beta4, alpha2beta1, antiintegrin alpha4, RANK-L, BLyS, c-MET, DR, DR10, TCRalpha, beta, ICOS, CTLA-4, CAIX (MN), EphA2, CA6, ovarian CA6, cervical CA6, breast CA6, angiopoietin-2, Cripto, ENPP3, Mesothelin, FOLR1, Nectin-4, TIM-1, Muc-16, Tissue Factor, LIV-1, GM2, alpha5 integrin, TLR-7, PD-1, AFP, CA125 (MUC16), Sialyl Lewis^{Y}, CAMPATH-1, HLA-DR, anti-idiotype, carcinoembryonic antigen (CEA), TAG-72, Folate-binding protein, A33, G250, gangliosides (including GD2, GD3, GM2), Le^{Y}, collagen 4 (collagen IV), collagen 18 (collagen XVIII), SC6, CA-125, CA19-9, p185^{HER2}, de2-7 EGFR, Fibroblast activation protein (FAP), Tenascin, metalloproteinases, Endosialin, Carbonic anhydrase, Galectin 9, Aldolase A, eIFgamma4, Tyrosinase, Galectin 4, HERKV-K10, p53, NY-LU-12, Restin, NY-CO-38, MAGE-1, MAGE-4a, SSX2, NY-ESO-1, SCP-1, HGFR, PTK 7, CCK-4, PDGFR, PTP-LAR, CDCP1, CADM1, IGSF4, Lu, BCAM, CEACAM6, JAM-A, PTGFRN (CD9P-1), MCAM, MUC18, MCP, EMMPRIN, TfR, TRAILR2, C1qR, hTERT, Survivin, MDM2, CYP1B1, Melan-A, MART-1, MART-2, Melanosomal proteins, gp100, neo-PAP, CDC27, MAGEs, WT1, MUM-1, MUM-2, MUM-3, BRAF, TPI, fibronectin, K-ras, beta-catenin, CDK4, caspase-8, p14^{ARF}, p16^{INK4a}, TGFβRII, bcr-abl, SYT-SSX, TRP-1, TRP-2, GnT-V, tyrosinase, FGF5, TEL-AML1, Proteinase 3, HER2/neu, AFP, MUC-1, EBV-EBNA, HTLV-1 tax, HPV16-E7, mutated HLA-A2, HA1, SART3, GnT-V, CEACAM5, AGS-16, GPNMB, ESAT-6, RANK, CanAg, fibrin, TF, PRAME, CA19-9, CA50, CA125, CA195, CAM17.1/WGA, AFP, beta-MG, DU-PAN2, HE4, b-2 microglobulin, transferrin, transthyretin, ApoA1, TROP-2, CTLA-4, GITR, PD-1, PD-L1, c-KIT, CD11b-CD18 integrin heterodimer, DNA/Histon H1, Folate, EpCAM, Tenascin-c, ECM (proteoglycan or fibronectin), fibrinogen, SV40 large T antigen, SC6-Ag, SC-Ag, DR4 (death receptor 4), DR5, ESA, mucin, hPAM4, hRS7, HLA-DR, or CCR4, antigen of general formula MTX1, MTX2, PECAM, thrombomodulin, Tn, cathepsin D, TYRO-3, MER
either the antibody or the antibody fragment is directed against a PF4/heparin complex.

7. Products of formula IB2 according to claim 1 for their use in the treatment of a tumor in particular colorectal cancer, hepatocarcinoma, a lung cancer, pancreatic cancer, non-small cell lung cancer, small cell lung cancer, colorectal cancer, mutated K-RAS colorectal cancer, a breast cancer, triple-negative breast cancer, metastatic breast cancer, liver cancer, head and neck cancer, Castleman disease, thyroid cancer, medulloblastoma, multiform glioblastoma, glioma, sarcoma, anaplastic astrocytoma, kidney cancer, stomach cancer, malignant ascites, prostate cancer (metastatic or not), solid tumors, acute myeloid leukemia, chronic lymphocytic leukemia, melanoma, myeloma, multiple myeloma lymphomas, mantle cell lymphoma, Hodgkin lymphoma, non-Hodgkin lymphoma, follicular lymphoma or for their use in the treatment of an inflammatory or autoimmune disease in particular graft rejection, rheumatoid arthritis, Crohn's disease, psoriasis, multiple sclerosis, periodic syndromes associated with cryopyrin, thrombocytopenic purpura.

8. Product according to one of the preceding claims, wherein the derivatives of the carboxylic acid function(s) is the amide formed with butanimide.

9. Product according to one of the preceding claims wherein M is the monomethylauristatin E or the monomethylauristin F.
